# EUROPEAN PATENT SPECIFICATION

(11) **EP 2 547 369 B1**
(45) Date of publication and mention of the grant of the patent: **19.04.2017**
(21) Application number: 11709651.1
(22) Date of filing: 21.03.2011
(51) Int. Cl.: A61K 49/18, A61K 9/107, A61P 35/00, B82Y 5/00

(54) **A NANOPARTICLE COMPRISING A MICELLE FORMED BY AN AMPHIPHILIC BLOCK-COPOLYMER AND ENCAPSULATING A GADOLINIUM COMPLEX**
NANOPARTIKEL MIT EINER ANHAND EINES AMPHIPHILEN BLOCKCOPOLYMERS GEBILDETEN MIZELLE ZUR VERKAPSELUNG EINES GADOLINIUMKOMPLEXES
NANOPARTICULE COMPRENANT UNE MICELLE FORMÉE PAR UN COPOLYMÈRE SÉQUENCÉ AMPHIPHILE ET ENCAPSULANT UN COMPLEXE DE GADOLINIUM

(30) Priority: 19.03.2010 PT 10501710
(43) Date of publication of application: 23.01.2013
(73) Proprietor: King Saud University, Riyadh 11451 (SA); Universidade do Algarve, 8005-139 Faro (PT)
(72) Inventor: MOUFFOUK, Fouzi, Riyadh 11451 (SA); RODRIGUES DOS SANTOS, Nuno, P-8005-139 Faro (PT); ALROKAYAN, Salman, A., H., Riyadh 11451 (SA); DA COSTA, Ana M. Rosa, Campus de Gambelas P-8005-139 Faro (PT); ABUSALAH, Khalid Mustafa, P.O. Box 2454 11451 Riyadh (SA)
(74) Representative: Engelhard, Markus
(86) International application number: PCT/EP2011/001392
(87) International publication number: WO 2011/113616

(56) References cited:
- WO-A2-01/87227
- JP-A- 2008 222 804
- KR-A- 20100 030 955
- US-A1- 2008 241 073
- GAO GUANG HUI ET AL: "Magnetite-nanoparticle-encapsulated pH-responsive polymeric micelle as an MRI probe for detecting acidic pathologic areas.", SMALL (WEINHEIM AN DER BERGSTRASSE, GERMANY) 6 JUN 2010 LNKD- PUBMED:20449849, vol. 6, no. 11, 6 June 2010 (2010-06-06), pages 1201-1204, XP002645298, ISSN: 1613-6829
- SETHURAMAN V A ET AL: "A biodegradable pH-sensitive micelle system for targeting acidic solid tumors", PHARMACEUTICAL RESEARCH 200803 US, vol. 25, no. 3, March 2008 (2008-03), pages 657-666, ISSN: 0724-8741
- KYUNG TAEK OH ET AL: "Cancer-associated pH-responsive tetracopolymeric micelles composed of poly(ethylene glycol)-b-poly(L-histidine)-b-poly(L-lacti c acid)-b-poly(ethylene glycol)", POLYMERS FOR ADVANCED TECHNOLOGIES JOHN WILEY & SONS LTD. UK, vol. 19, no. 12, December 2008 (2008-12), pages 1907-1913, ISSN: 1042-7147
- ZHAO H ET AL: "Folate-conjugated polymer micelles with pH-triggered drug release properties", MACROMOLECULAR RAPID COMMUNICATIONS - SYNTHETIC BIOMACROMOLECULES FOR DELIVERY OF THERAPEUTICS, TISSUE ENGINEERING, AND IMAGING 20100701 WILEY-VCH VERLAG DEU, vol. 31, no. 13, 1 July 2010 (2010-07-01), pages 1163-1169, DOI: 10.1002/MARC.200900876
- LEE ET AL: "Tumor pH-responsive flower-like micelles of poly(l-lactic acid)-b-poly(ethylene glycol)-b-poly(l-histidine)", JOURNAL OF CONTROLLED RELEASE, ELSEVIER, AMSTERDAM, NL, vol. 123, no. 1, 25 September 2007 (2007-09-25), pages 19-26, XP022265747, ISSN: 0168-3659, DOI: 10.1016/J.JCONREL.2007.08.006

## Description

### ABSTRACT OF THE DISCLOSURE

The present invention relates to a nanoparticle comprising a micelle formed by an amphiphilic block-copolymer and an agent encapsulated within said micelle. The present invention also relates to a composition comprising such nanoparticle and to the use of such nanoparticle and/or of such composition. More particularly, in one embodiment, the invention describes a new class of polymeric nanoparticles as smart T1 contrast agent for MR imaging for breast cancer early detection. These nanoparticles contrast agents have the capability to remain switched off during circulation and then switch on their imaging capacity upon arrival at the target sites (tissue of interest). These smart nanoparticles contrast agent are self-assembled from pH sensitive amphiphilic polymer, loaded with Gadolinium (Gd³⁺) complex based T1 agent and then fitted with targeting biomolecules such as antibody, small molecules or DNA to increase its specificity toward the target of interest.

### BACKGROUND OF THE INVENTION

The internal depths of the human body have long been an uncharted "dark world", as visible light is unable to penetrate the tissues. Although observation of the body cavity is possible by the use of surgical procedures, it would be preferable to observe the inner spaces of the body by noninvasive means. The relatively recent developments of imaging techniques such as computed tomography (CT), near-infra-red (IR) fluorescence imaging, positron emission spectroscopy (PET), and magnetic resonance imaging (MRI) have made such exploration possible, and has enabled mankind to make major advances in the field of biomedical diagnosis and therapy.

As most biological processes and diseases are related to molecular and cellular events, the precise observation of these detailed biological processes is important [1]. However, classical imaging techniques are generally unsatisfactory for such "molecular imaging" applications, and the development of high-performance imaging systems capable of identifying detailed biological processes at the molecular and subcellular levels is important. Currently, MRI is one of the most powerful medical diagnostic tools available, due mainly to its noninvasive nature and multidimensional tomographic capabilities, coupled with high spatial resolution [13]. Although in terms of sensitivity MRI lags behind other tools [2]. Toword this end several attempts have been made to improve the sensitivity of MR imaging using classic means, but with the increasing ability of nanotechnology to create many devices at a the cellular and molecular scale, nanotechnology is expected to lead to major advances in medical imaging especially in MRI (T2), examples include superparamagnetic iron oxide (SPIO) and Magnetodendrimers nanoparticles (NP). Where these bioconjugated nanoparticles act as imaging probes, which can efficiently report on various molecular/biological events occurring in region of interest. Molecular MR studies utilizing such iron oxide-biomolecule conjugates include the imaging of inflammation [3], infarction [4], angiogenesis, [5], apoptosis [6], gene expression [7], b-amyloid plaques [8], and cancer [9]. Although much progress has been made in the development of magnetic NP T2 contrast agents for molecular MR imaging during the past few years, their successful use is limited to in-vitro systems, except for a few in-vivo cases. Two main difficulties lie in the poor MR contrast effects and limited stability and biocompatibility under in-vivo conditions. The MR signal-enhancing effect of conventional iron oxide-based NPs is unsatisfactory compared to other diagnostic tools such as fluorescence and PET. Hence, it was an object of the present invention to avoid the afore-mentioned problems and disadvantages.

WO 01/87227 A2 discloses polymeric micelles which are pH and/or temperature sensitive, and which are used to increase potency of therapeutic agents, including anti-tumor drugs. US 2008/0241073 A1 discloses contrast agents for MRI which comprise as an effective ingredient a polymeric micelle having gadolinium (Gd) atoms in an inner core and an outer shell including hydrophilic polymer chain segments, which micelle is delivered to a tissue(s) and/or site(s) of solid tumor(s) *in vivo.* The contrast agents comprise a GD-bound block copolymer. JP 2008 222804 discloses polymer micelle MRI contrast agents which are formed from a block copolymer comprising a hydrophilic polymer chain segment and a Gd-containing segment. Thereby, the Gd-containing segment has a plurality of side chains with amino groups, wherein Gd chelating agents are bound to some or all of the amino groups, either directly or via linker structures.

The objects are solved according to the invention as it is claimed in the claims.

Accordingly, in a first aspect, the present invention relates to a nanoparticle comprising a micelle formed by a pH sensitive amphiphilic block-copolymer, said pH sensitive amphiphilic block-copolymer being poly(ethylene glycol-b-trimethylsilyl methacrylate), and an agent encapsulated within said micelle, said agent being a Gd³⁺-complex.

In one embodiment, said pH sensitive amphiphilic block-copolymer is capable of forming said micelle in aqueous solution through self-assembly.

In one embodiment, said nanoparticle is conjugated to a targeting agent capable of binding specifically to a target cell.

In one embodiment, said Gd³⁺-complex is selected from the group comprising tetraaquodichloro(4,4'-ditBu-2,2'-bipyridine)gadolinium (III)chloride, or any other Gd³⁺-complexes such as **DO3A** 1,4,7,10-tetraazacyclododecane-1,4,7-trisacetic acid, **DO3AB** 1,4,7,10-tetraazacyclododecane-1-(2,3-dihydroxy-1-hydroxymethylpropyl)-4,7,10-trisacetic acid, **DO3A(tris-*t*-Buester)** 1,4,7-tris-*tert*-butoxycarbonylmethyl-1,4,710-tetraazacyclododecane, **DOTA** 1,4,7,10-tetraazacyclododecane-1,4,7,10-tetraacetic acid, **DOTA-*p*-NH2-anilide** 1,4,7,10-tetraazacyclododecane-1-(4-aminophenylcarbamoylmethyl)-4,7,10-trisacetic acid, **DOTA(tris-*t*-Buester)** 1,4,7,10-tetraazacyclododecane-1,4,7-tris(acetic acid-*tert*-butyl ester)-10-acetic acid, **DPDP** dipyridoxal diphosphate, **DTPA** diethylenetriaminepentaacetic acid N **DTPA-BMA** {bis-[2-(carboxymethylmethylcarbamoylmethylamino) ethyl]amino} acetic acid **DTPA-BMEA** [bis-(2-{carboxymethyl-[(2-methoxyethylcarbamoyl)methyl]amino}ethyl)-amino]acetic acid, **EOB-DTPA** {[2-(biscarboxymethylamino)-3-(4-ethoxyphenyl)propyl]-[2-(biscarboxymethylamino) ethyl]amino}acetic acid.

In one embodiment, said targeting agent is selected from the group comprising (a) proteins, such as natural proteins, recombinant proteins, antibodies, antibody fragments, lipoproteins and protein ligands, such as growth factors, e.g. EGF, GM-CSF, VEGF, TGF, and chimeras thereof, cytokines, e.g. interleukins, interferons, transferin, adhesion molecules, e.g. CD4, and urokinase-type plasminogen activator, (b) peptides, such as natural peptides and synthetic peptides, (c) nucleic acids, such as natural or designed DNA, RNA or derivatives thereof, (d) carbohydrates, (e) lipids, (f) hormones, and (g) chemical compounds.

In one embodiment, said target cell is selected from the group comprising cancerous cells, parasitic cells, cells infected by a virus and cells releasing TNFα.

In one embodiment, said micelle is stable at neutral pH and disintegrates at a pH < 6.

In one embodiment, said micelle is capable, preferably after chemical modification, to release its content encapsulated therein either due to enzymatic reaction(s), due to the presence of metals, such as Ca and Zn, or due to exposure to light of a defined wavelength or wavelength range.

In a further aspect, the present invention relates to a composition comprising nanoparticles according to present invention, wherein said agent that is encapsulated within said micelles of said nanoparticles is a Gd³⁺-complex.

In one embodiment, said agent encapsulated within said micelle is a Gd³⁺-complex, and said composition or nanoparticle according to the present invention is for use as a magnetic resonance imaging contrast agent, in particular for detection of cancer, or for use to treat a disease, preferably cancer, or a disease that is characterized by a specific microenvironment within the tissue affected by such disease, said specific microenvironment including parameters, such as pH being above or below physiological pH, the presence or absence of specific enzymes or metals, metal anions and/or cations, or a defined temperature above or below the physiological temperature.

It should be noted that the composition or the nanoparticle according to the present invention, when being used as a magnetic resonance imaging contrast agent has the capability to remain switched off as a contrast agent, whilst being in circulation within the body/organism, and to switch on its imaging contrast agent capability upon arrival at target site(s), i.e. the intended target cells, where it releases the agent encapsulated within the micelles to or into the target cells.

It should also be noted that the composition and nanoparticle according to the present invention, when used as a magnetic resonance imaging contrast agent is capable to detect cancer at a very early stage, as an example at the first two second stage of cancer. Typically, in the first stage of cancer, the cancer is localized to one part of the body, whereas in the second stage, the cancer is locally advanced.

In one embodiment, said composition or nanoparticle is administered to a patient in whom a magnetic resonance imaging contrast agent is intended to be used, or is administered to a patient in need thereof, e.g. a patient having or suspected of having a disease, e.g. cancer.

In one embodiment, the amphiphilic block-copolymer forms the micelle in aqueous solution, typically through a process of self-assembly. The term "self-assembly" as used herein, is meant to refer to a process in which the amphiphilic block-copolymer forms an organized structure, in this case a micelle through the interaction of the hydrophobic blocks with each other, thus forming the core of micelle, whereas the hydrophilic blocks stay in contact with water form the outside of the micelle (also sometimes referred to as the "corona"). The term "the nanoparticle is conjugated to a targeting agent" is meant to refer to a linkage between the nanoparticle and the targeting agent which may be covalent or non-covalent. For example, part of the targeting agent may have become incorporated in the micelle, with a portion of the targeting agent sticking out of the micelle and thus allowing and enabling its targeting action.

In one embodiment, the targeting agent is an agent that specifically binds to a target cell and, because it is conjugated to the micelle in accordance with the present invention, it will lead to the micelle being targeted to the target cell.

In one embodiment, the nanoparticle in accordance with the present invention consists essentially of the micelle formed by an amphiphilic block-copolymer and said agent which is encapsulated within said micelle. Optionally, said targeting agent may be conjugated to said micelle.

In a preferred embodiment, the targeting agent is an antibody or an antibody fragment or a growth factor, cytokine or transferrin. In one embodiment, the target cell comprises a component which is recognized by the targeting agent. Such component may for example be a growth factor receptor, a cytokine receptor, a transferrin receptor, cluster of differentiation (CD) antigens, Lewis antigens, MUC1-antigen, or Wilms'tumor gene product. In one embodiment, the target cell is a cancerous cell. The nanoparticles in accordance with embodiments of the present invention can be used to be specifically directed at cancer cells for early detection purposes. During circulation of the nanoparticles according to the present invention through the body, the content of the micelle remains encapsulated (switch off state) and is only delivered once the nanoparticle/micelle has reached the target, i.e. the cancer cell, because of a different pH (switch on state). Thus, the content of the micelle is released and can exert its function, for example it can exert its toxicity towards the cancer cell or it can act as an MRI-contrast agent. Consequently, the nanoparticles in accordance with the present invention can also be used to treat diseases. In one embodiment, they are used to treat cancer.

The present invention also relates to a composition comprising the nanoparticles according to the present invention. Additionally such composition may comprise an aqueous solution wherein the nanoparticles are dissolved or dispersed. The present invention also relates to the use of such composition or of such nanoparticle as a contrast agent in magnetic resonance imaging (MRI). The present invention also relates to the use of such nanoparticles or composition for use in a method of treating a disease. The present invention also relates to a method of treatment of a disease, comprising administering nanoparticles in accordance with the present invention or a composition in accordance with the present invention to a patient in need thereof having such disease, preferably, the disease is cancer, more preferably breast cancer.

The present inventors have devised means to be able to deliver in a targeted manner and target-specific manner an agent to a site where the action of such agent is intended.

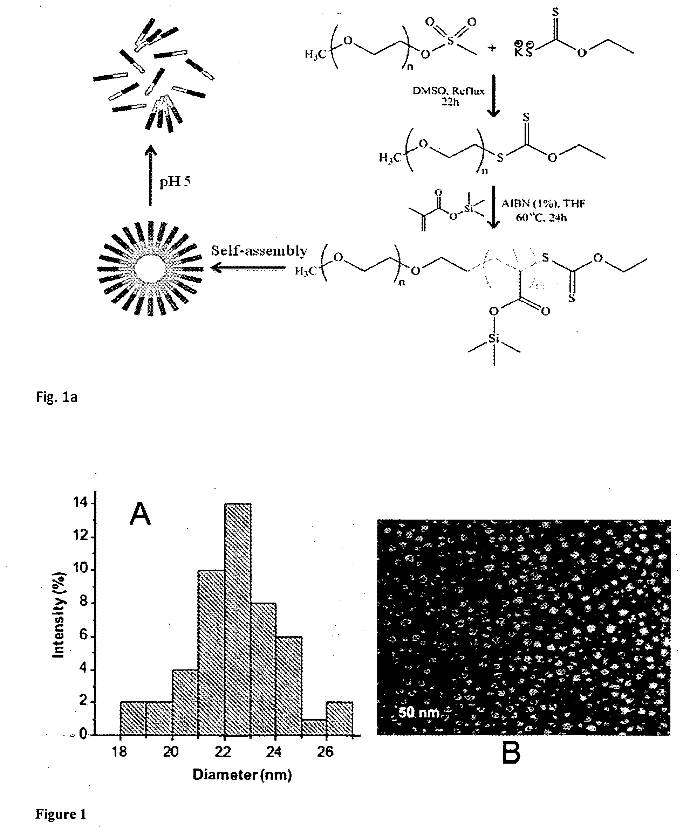

Toward this aim, in one embodiment, a new class of bioconjugated pH sensitive polymeric nanocarriers loaded with pH sensitive Gd³⁺ based T1 agent has been designed and prepared (see figure 1). The proposed nanoparticles based contrasts, have the ability to remain switched off during circulation and then switch on their imaging capability upon arrival at the target sites. The functional approach of the proposed relaxometric MR probes is the following: A contrast agent usually is a chelated Gd³⁺ complex that enhances MR images by decreasing the longitudinal relaxation time (T1) through interaction of the paramagnetic lanthanide with surrounding water protons. Therefore restricting water accessibility to those complexes via their encapsulation within the hydrophobic core of the polymeric nanoparticle prevents them from decreasing the T1 of the nearby water molecules; as a result the contrast will remain switched off. However when these chelated Gd³⁺ complexes are released to the medium, they immediately turn on their contrast ability provoking a significant increase in the signal intensity at the tumor sites. The release mechanism that has been introduced within these nanocarriers explores the cancer unique microenvironments such as acidic pH, therefore the present inventors prepared new class of nanoparticles that disintegrate at a precise pH (cancer tissue pH) leading to the contrast release. To avoid all unsolicited contrast activity that might occur by unexpected breakup of the nanocarriers outside the target sites, a pH sensitive Gd³⁺ contrast agent is to be used as a second layer of protection. Therefore, the contrast agent will remain switched off outside the target sites until the end of the test.

### The nanoparticles according to the present invention have the following advantages:

1- The newly developed nanoparticles contrasts have the ability to remain switched off during circulation and then switch on their imaging capability upon arrival at the target sites.
2- A New method to suppress the imaging capability of Gd³⁺ complexes in order to control the longitudinal relaxation time (T1) of water molecules in the tissue of interest.
3- Improve the Magnetic Resonance Imaging (MRI) sensitivity of this imaging technique for early cancer detection and other types of cancer and diseases.
4- The functional approach of the new relaxometric MR probes is the following: Contrast agents, usually is a chelated Gd³⁺ complex that enhances MR images by decreasing the longitudinal relaxation time (T1) through interaction of the paramagnetic lanthanide with surrounding water protons. Therefore restricting water accessibility to those complexes via their encapsulation within the hydrophobic core of the polymeric nanoparticle prevents them from decreasing the T1 of the nearby water molecules; as a result the contrast will remain switched off. However when these chelated Gd³⁺ complexes are released to the medium, they immediately turn on their contrast ability provoking a significant increase in the signal intensity at the tumor sites.
5- The release mechanism that has been introduced within these nanocarriers explores the cancer (or other diseases) unique microenvironments factors that might underlie the stability of these polymeric nanoparticles such as pH, enzymes. Therefore the present inventors prepared a new class of nanoparticles that disintegrate at a precise pH (cancer tissue pH) leading to the contrast release.
6- Increase the half-life in the blood of the contrast agent by providing a protection from the outside elements (in vivo environment) via their encapsulation within the core of these nanoparticles.
7- Replacement of the random accumulation of the contrast in tissue of interest by a real targeting capability that has been harnessed via the targeting agent of these polymeric nanoparticles.
8- Increase the sensitivity through signal amplification of the targeted tissue. The signal is amplified via the massive amount of the contrast agent that has been encapsulated within these nanoparticles. This last feature allows us to detect cancer in early stage.
9- Decrease the time of MRI test.
10- MRI has a high incidence of false-positive test results that indicate cancer is present when it is not (compare with x-ray mammography and positron emission mammography **PEM**). Researchers believe these false positives are due in part to hormonal changes that occur during a woman's menstrual cycle and breast tissue texture. Therefore using this new method will increase considerably the sensitivity of MRI toward cancer early detection.
11-Interpreting MR images is also extremely difficult and requires a lot of years of practice, consequently using this new type of contrast will reduces the risk of miss reading. Because if the patient is free of disease the doctor will not see any signal, but in the opposite case huge bright spots will appear at the target site.
12- Reduce the amount of contrast used in MRI by 50 folds
13- Create a smart contrast agent with dual functions; detecting cancer and killing it at the same time, which will be one of its kinds among contrast agents.
14- This new smart contrast agent can be used to detect other type of cancer and diseases.
15- A new class of pH sensitive polymeric nanoparticles contrast agent for cancer early detection, using MRI.
16- The amount of gadolinium chelates used in the MRI test is greatly reduced.
17- The time of the test is greatly reduced.
18- The contrast in the MR images is enhanced via signal amplification provided by the large number of contrast agent encapsulated within the nanoparticles.
19- Smart polymeric nanoparticles
20- The first MRI smart contrast agent that can detect and kill in the same time cancer at early stage at the same time.
21- Makes MR images easy to interpret even for inexperienced doctors.

Furthermore reference is made to the figures wherein the figures show the following:

### LIST OF FIGURES

**Figure 1a****:** Synthetic approach for the synthesis of poly(ethylene glycol-*b*-trimethylsilyl methacrylate) and schematic representation of its self-assembly into micelles and their disaggregation.
**Figure 1****: (A)** Dynamic Light Scattering results for loaded polymeric micelles after the encapsulation of the dye. **(B)** TEM of dye loaded polymeric micelles after the self assembly of poly(ethylene glycol-*b*-trimethylsilyl methacrylate), bar scale 50nm.
**Figure 2****:** Fluorescence spectra of 1-methylpyrene monomer (375 nm) and excimer (480 nm) at pH=7 when polymeric micelles are stable, and their disassembling at pH=5 with parallel dye release and consequent augmented and unique monomer emission.
**Figure 3****:** Relaxometric experiment that measure the T1 before and after the release of the contrast from the polymer nanoparticle using NMR.
**Figure 4****:** MRI experiment using mimic healthy and cancer tissue A) mimic healthy tissue, B) mimic cancer tissue.

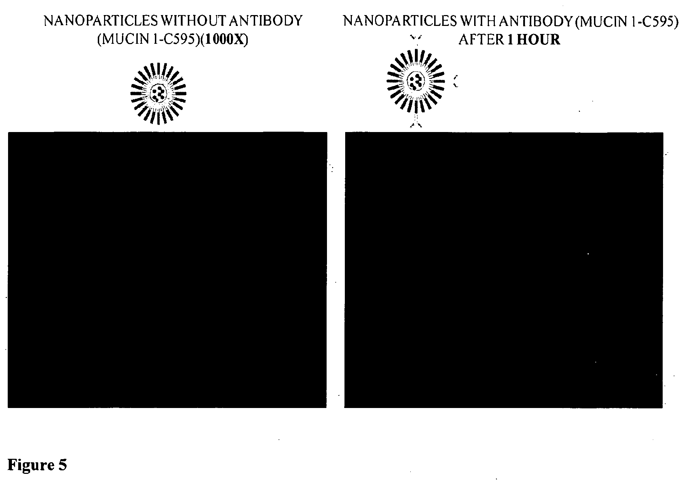

**Figure 5****:** The ability of targeting cancer cells (MCF-7 breast cancer cell line) by the newly developed polymeric nanoparticles
**Figure 6****:** MRI experiment performed on mouse A) injected with lactid acid and then with pH sensitive polymeric nanoparticles loaded with Gd³⁺ contrast agent B) mouse injected only with pH sensitive polymeric nanoparticles loaded with Gd³⁺ contrast agent.
**Figure 7****:** Toxicity studies of the free Gd+ contrast and Gd+ contrast encapsulated within the pH sensitive polymeric nanoparticles.
**Figure 8****:** Cytotoxicity of free Gd(III) complexes by cell count. Comparison of cytotoxicity of varying concentrations of Gd(III) complexes using varying concentrations of Gd(III) complexes using cell count with Trypan Blue on Jurkat cell line, during 3 days. Data represent the means of duplicate ± standard deviation.
**Figure 9****:** Cytotoxicity of free Gd(III) complexes by MTT assay. Comparison of cytotoxicity of varying concentrations of Gd(III) complexes using MTT assay on MCF-7 (A) and Jurkat (B) cell lines. The test was performed at the end of 3 days of treatment. Data represent the means of eight testes + standard deviation.
**Figure 10****:** Linear regression line for cytotoxicity of free Gd(III) complexes. A linear regression line was calculated from data obtained by MTT assay, to be used as a standard curve to find the concentrations of Gd(III) complexes to reduce cell viability to 50% of control values for MCF-7 (A) and Jurkat (B) cell lines.
**Figure 11****:** Cytotoxicity comparison of free and nanoparticle- encapsulated Gd(III) complexes. Comparison of cytotoxicity of varying concentrations of free and nanoparticle-encapsulated Gd(III) complexes using MTT assay on Jurkat human leukemia cell line, at the end of a incubation period of 3 days. Data represent the means of quadruplicate + standard deviation.
**Figure 12****:** Detection of *MUC1* expression by RT-PCR. A MUC1 was expressed by all used cell lines; though the highest expression was shown by MDA-MB-468 cells. DNA marker (100-600 bp ladder).
**Figure 13****:** *MUC1* relative expression, in different cell lines, by real-time PCR analysis. The highest *MUC1* expression was shown by MBA-MB-468 human breast adenocarcinoma cell line, when compared to other examined cell lines.
**Figure 14****:** Electrophoresis and detection of MUC1 glycoprotein in homogenates of MCF-7 breast cancer cells and DND41 acute lymphoblastic leukemia cells by Western Blotting. The reaction with anti-MUC1 antibody confirmed the presence of MUC1 in MCF-7 homogenates.
**Figure 15****:** Analysis by fluorescent flow cytometry to detect MUC1 cell surface expression on MCF-7 and MDA-MB-468 cell lines. MUC1 expression was detected on both cell lines using the ani-MUC1 extracellular domain, C595 mAb (black line). The discontinuous black line indicates reactivity with an irrelevant antibody of the same isotype. The grey shaded region is the control group.

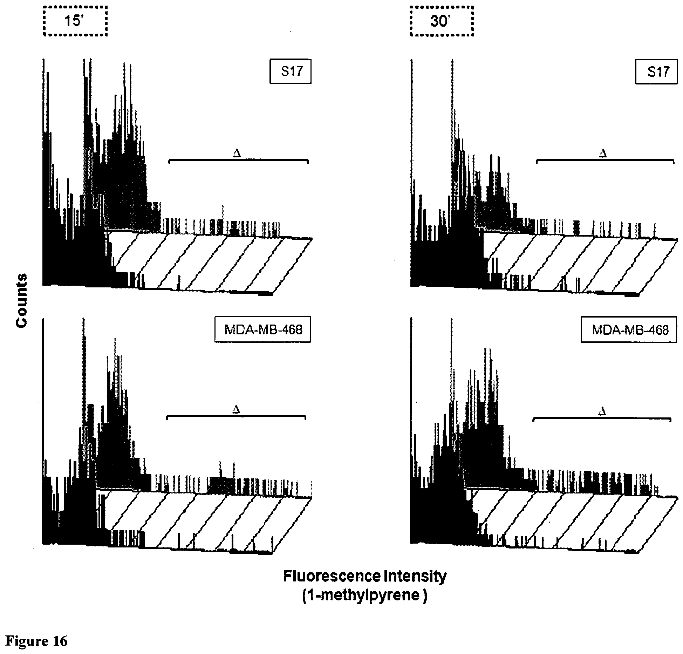

**Figure 16****:** Fluorescent flow cytometry to assess specific targeting of Anti-MUC1 bioconjugated 1-methylpyrene loaded nanoparticles. An enhancement in fluorescence intensity is evident on MDA-MB-468 cells which were incubated for 15 minutes and for 30 minutes with Anti-MUC1 bioconjugated nanoparticles (gray shaded region) comparing with S17 cells. The black shaded region indicates the fluorescent enhancement due to cell incubation with non-targeted 1-methylpyrene loaded nanoparticles. Delta symbol (Δ) designates the fraction of cells that showed a fluorescent intensity enhancement due to uptake of 1-methylpyrene-loaded nanoparticles. Delta values are presented on table 2.
**Figure 17****:** Specific binding of anti-MUC1 1-methylpyrene-loaded micelles to MCF-7 breast cancer cells. At the end of an incubation period of 1 hour, it was evident the differential uptake of non-targeted and anti-MUC1 micelles by MCF-7 cells.
**Figure 18****:** Specific binding of anti-MUC1 1-methylpyrene-loaded micelles to MCF-7 breast cancer cells, after washes. After washing 3 times high 1-methylpyrene fluorescence was found in cells incubated with anti-MUC1 polymeric micelles, while a low level of background fluorescence was visible in MCF-7 cells incubated with non-targeting micelles.

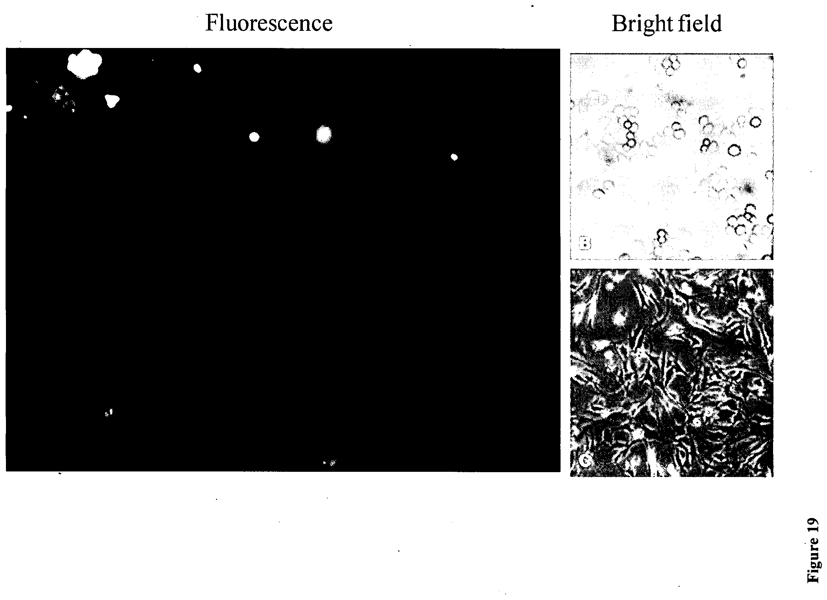

**Figure 19****:** Increased targeting of anti-MUC1 1-methylpyrene-loaded micelles to MDA-MB-468 breast cancer cells. MDA-MB-468 (B) and S17 (C) cells were seeded with the proportion of 1:5 and incubated for 1 hour with anti-MUC1 bioconjugated micelles. After incubation and cell fixation, it was evident the increased uptake of anti-MUC1 micelles by the human MUC1-expressing MDA-MB-468 cells (A). So by doing this, the present inventors observed that MDA-MB-468 cells (rounded morphology) Figure 19B, incorporated more 1-methylpyrene fluorescence (Figure 19A) than S17 cells (elongated morphology) Figure 20-C. Indeed, round (MDA-MB-468) cells presented bigger and brighter spots than elongated (S17) cells in the mixed cultures (Figure 19A).

### Detailed description of embodiments of the invention

Polymeric micelles have gained a lot of attention in the field of drug delivery due to their great stability and capability of encapsulation [15]. Polymeric micelles are formed by self-assembling amphiphilic block-copolymers when they are dissolved in selective solvents [16]. Upon micellization, the hydrophobic blocks interact with each other and form the core of the micelle while the hydrophilic blocks stay in contact with water to form the corona [17]. The shape and the size of the polymeric micelles are influenced by: 1) the copolymer chains size and chemical nature. 2) The type of solvent used to dissolve the copolymer and 3) the critical micelle concentrations of the copolymer. Since these polymeric nanoparticles (or micelles) have proven to be stable and efficient carriers for hydrophobic drug molecules, the present inventors managed to encapsulate Gd³⁺ complex-based contrast agents inside these bioconjugated nanoparticles. The newly developed polymeric nanoparticles based contrasts, will have the ability to remain switched off during circulation and then switch on their imaging capability upon arrival at the target sites (cancer tissue). By restricting the water access to the Gd⁺³ complexes that are located inside the micelles, the relaxivity of water molecules remain unchanged, however when these Gd⁺³ complexes are released from the micelle the Gd⁺³ complexes recover its ability and reduce the T1 of the water surrounding the targeted tissue. The specificity of these polymeric nanoparticles based contrasts will be fitted with specific antibody that recognizes the target tissue.

Moreover reference is made to the following examples which are given to illustrate the present invention.

### Example 1

The pH-sensitive polymeric nanoparticle according to the present invention, is able to encapsulate a large number of contrast molecules during its self-assembly from simple building blocks (e.g. Poly(ethylene glycol-b-trimethylsilyl methacrylate). The sensitivity of these polymeric nanoparticles to pH is due to their silicon moieties that can be cleaved under mildly acidic condition, rendering the polymer hydrophilic, which leads to disintegration of the colloidal particles causing the load release. In one embodiment, Poly(ethylene glycol-b-trimethylsilyl methacrylate) was synthesized by Reversible Addition-Fragmentation chain Transfer (RAFT) polymerization from trimethylsilyl methacrylate (TMSMA) using α-(O-ethylxanthate)-ώ-methylPEG 2'000 as macro-Chain Transfer Agent (macro-CTA) and a monomer to macro-CTA ratio of 45:1. Azobisisobutyronitrile, AIBN (1 mol% of the monomer), was used as radical initiator [18] figure la. The contrast agent was synthesized and characterized via sample and Wesll known synthesis routes to generate hydrophobic gadolinium (III) complexes with ligands such as bipyridine. [19].

The preparation of bioconjugated polymeric nanoparticles and contrast encapsulation were carried out using a modified reported procedure [20]. In brief, poly(ethylene glycol-*b-*trimethylsilyl methacrylate) and the fluorophore (1-methylpyrene) or the contrast complex were dissolved in DMF. The mixture was stirred for 3h at room temperature. Subsequently, 2 ml aqueous solution containing C595-PEG (anti-MUC1) were added at a slow rate to induce the micellization. The resulting solution was then placed in a dialysis cassette with a 10-kDa MWCO and dialyzed against deionized water for 2 days at 4°C. The size distribution profile of the novel dye-encapsulated bioconjugated micelles was found to be around 24 nm (Figure 1A), as measured by dynamic light scattering (DLS). These micelles were also visualized under transmission electron microscopy (Figure 1B) by negatively staining with 1% uranyl acetate, and the size matches the values derived from DLS studies.

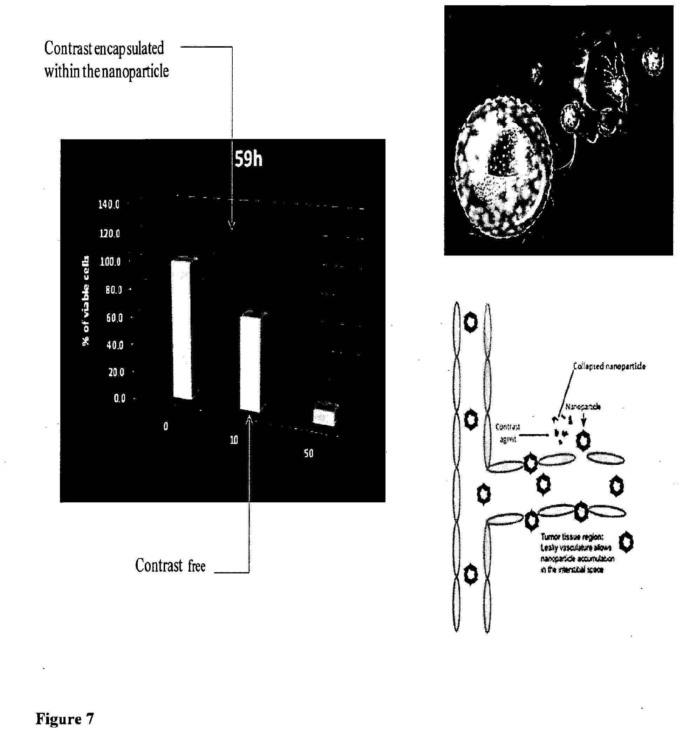

Proof of encapsulation and cytotoxicity study; Gd3+ complexes prepared by our group are potentially toxic, the present inventors determined how toxic Gd³⁺ complexes are for human cells and whether encapsulation in polymeric micelles could reduce their toxicity. To test cytotoxicity the present inventors used two type of cells, the Jurkat human leukemia cell line and MCF-7 breast cancer cell line. Culture of MCF-7 or Jurkat cells for 3 days in the presence of increasing concentrations of Gd3+ complex resulted in a dose-dependent loss of viability, as measured by the MTT assay. To determine the concentration of Gd³⁺ complex able to kill 50% of cells (IC50), a linear regression line was plotted for MCF-7 and Jurkat cells. Thus, the Gd³⁺ IC50 was 12.8 µM for MCF-7 and 10.2 µM for Jurkat. Next, the toxicity of Gd3+ complex encapsulated in micelles was assessed for MCF-7 cells. Fifty µM of Gd³⁺ complex induced overall cell death after 3 days. However, this toxicity was dramatically diminished when similar concentrations of Gd³⁺ complexes were encapsulated within polymeric micelles. These results indicate that Gd3+ complexes were effectively encapsulated inside the micelles and that this prevented their toxic effects to cells. Also these results suggest the possibility of using these smart contrast agents as a drug carrier for cancer treatment. When the cancer is detected and the contrast is released, the same contrast can act also as drug to kill cancer cells via the Gd³⁺ complex increased toxicity, in this way the present inventors create a contrast is with dual functions; detecting cancer and killing it at the same time figure 7.

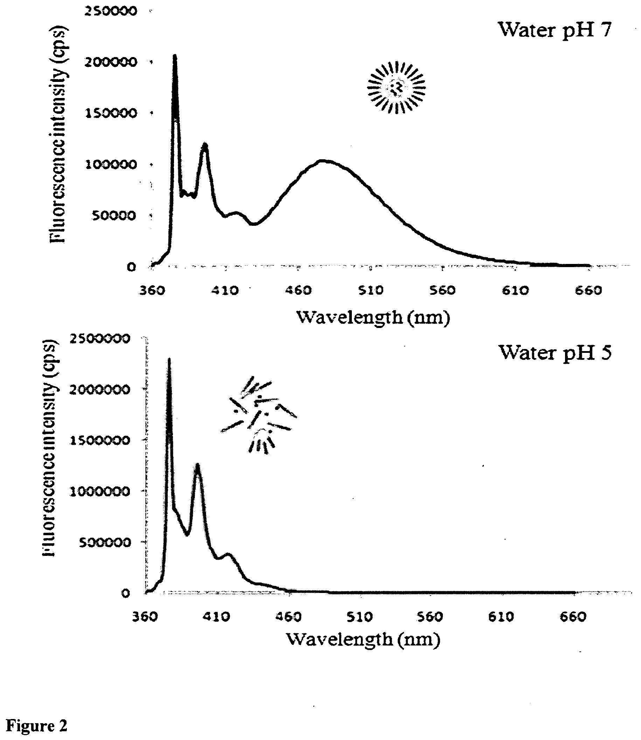

To establish the amphiphilic aggregation of the copolymer and its disaggregation, with parallel tracer release under pH change, the present inventors used the 1-methylpyrene fluorescence spectrum. This pyrenyl moiety displays simpler spectra than pyrene, with an intense polarity independent band for monomer emission (375 nm), has analogous low solubility in aqueous media (ca. 10⁻⁷ M), and still retains high quantum yield and rather long lifetime in micelles. Fluorescence probing of amphiphilic assemblies involves the measurement of changes in the emission properties of probes and/or photophysical intermediates (excimers or exciplexes). Pyrene excimer formation is a well-known concentration-dependent process in organic solvents. In sufficiently dilute solutions, only the excited monomer (P*) emission occurs. However, when pyrenyl concentration exceeds a threshold value (≈10⁻⁴ M), excimer (E*) formation can take place in two different ways: dynamically, through diffusional encounters of one P* with one ground-sate pyrenyl (P), or static formation may occur in pyrene-labeled polymers due to ground-state complex formation in aqueous media. Given the low solubility of pyrene in aqueous media, the excimer is not observed in pure water solution. But since apolar pyrenyls partition readily into hydrophobic media, these dyes molecules are effectively concentrated inside surfactant assemblies. The observation of both monomer (at 375 nm) and excimer (480 nm) bands establishes therefore the unequivocal occurrence of amphiphilic structures in aqueous media. Conversely, in the case of particle decomposition, only monomer emission is noticed, due to dilution of pyrenyl below the excimer-forming concentration threshold, as the copolymer and dye molecules solubilize. Figure 2 presents the spectroscopic proof of polymeric micelles formation at pH=7 and their subsequent disaggregation with parallel release of 1-methylpyrene at pH=5.5: at pH=7 the polymeric micelles are stable, as the excimer band holds, and at pH=5.5 only the monomer emission stands due to complete release of the dye.

After proving the ability of these particles to release its load in acidic condition, the present inventors emphasized that the encapsulated hydrophobic Gd³⁺ complexes within the core of these polymeric nanoparticles, will shield them from water molecules surrounding the micelles, consequently the T1 of aqueous solution surrounding these nanoparticles will remained unchanged (switch off state). However, when these nanoparticles are exposed to low pH similar to those found in cancer tissue, they disintegrate releasing the contrast causing the decrease in the T1 of the aqueous solution surrounding these nanoparticles (switch on state). The acidity of the cancer tissue is attributed to the extensive production of lactic acid within tumor cells and its extrusion to the interstitial fluid where the acid is accumulated due to an impaired clearance.

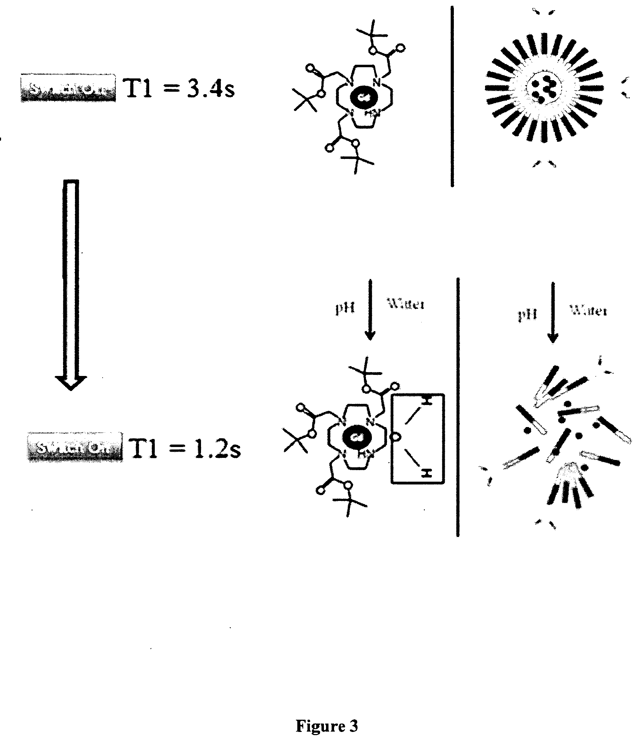

To prove the concept a series of experiments have been carried out. pH sensitive polymeric nanoparticles that have been loaded with contrast agent (tetraaquodichloro(4,4'-ditBu-2,2'-bipyridine)gadolinium(III) chloride) has been prepared and then added to aqueous solution (pH 7). First the T1 of pure water was measured using 1.5 T MR NMR scanner (nuclear magnetic resonance) and found to be 3.7s, then the T1 of the solution that contain the pH sensitive polymeric nanoparticles loaded with contrast agent at pH 7 was also measured and found to have exactly the same value of T1 which is 3.7, which means that the smart contrast is inactive or switched off. However, when the pH of the mixture was dropped from 7 to 5 the present inventors observed a net decrease in T1 of water molecules from 3.7s to 1.2s and that indicates the recovery the imaging capability of the contrast agent (switch on state) Figure 3.

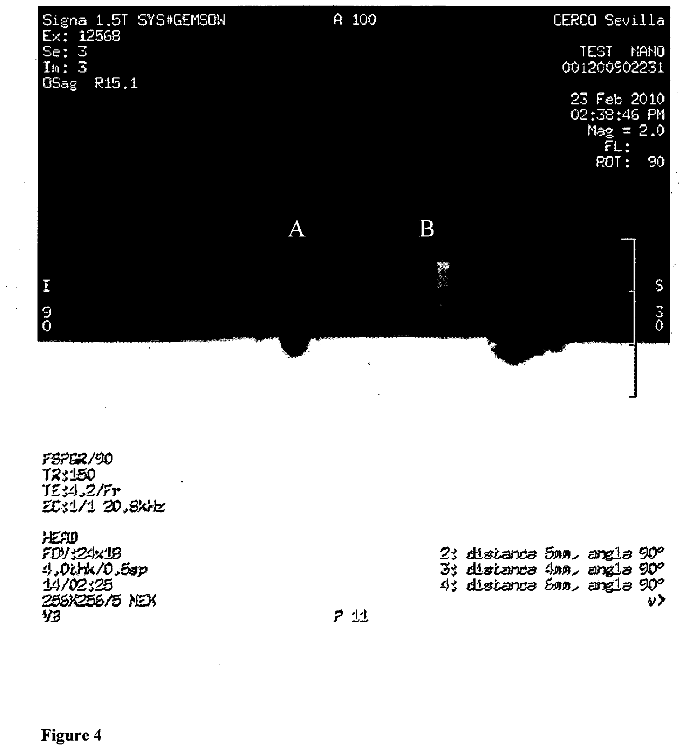

Also to demonstrate furthermore the concept, the present inventors arranged another experiment but this time using MRI equipment for patients (GE EchoSpeed 1.5 MRI). In this experiment the present inventors prepared two samples in two separate plastic tubes, the first one simulate cancer tissue with low pH around 5.4 and the second one a healthy tissue with pH around 7, afterward both samples were injected with the same pH sensitive polymeric nanoparticles loaded with the contrast agent and then an image was taken using MRI (figure 4), in this figure one can clearly see the difference. In sample (1) that mimics the cancer tissue one can see a bright white spot indicating the activation of the contrast agent imaging capability (switch on state). However in sample (2), that represents the healthy tissue remains dark with no signal, indicating that the contrast remained switch off due to the lack of the target tissue environment. Both experiments prove without doubt the ability of these smart polymeric nanoparticles based MRI contrast, to remain switched off during circulation and then switch on their imaging capability upon arrival at the target sites.

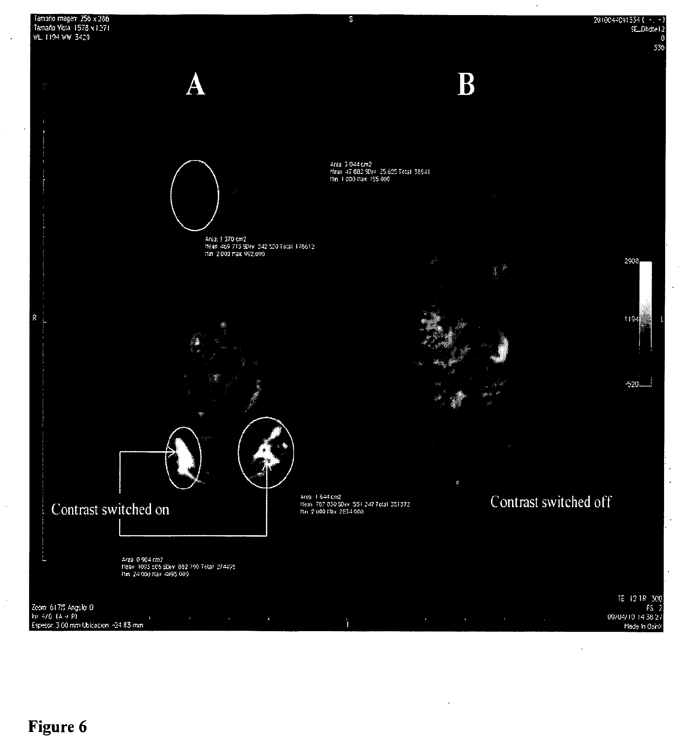

The third experiment that the present inventors conducted *in vivo* to prove the concept of our smart contrast agent consisted of testing these smart particles on mice. Two healthy mice were used, the first mouse was injected in his legs muscles with saline acidic solution to simulate cancer tissue at this location and the second mouse was left without any injection. After that both mice were injected with the pH sensitive polymeric nanoparticles loaded with Gd³⁺ complex in each leg of both mice with an amount of 0.25 ml which correspond to approximately 9 µg of Gd³⁺ complex (contrast agent). Then both mice were imaged using MRI (GE EchoSpeed 1.5 MRI). As depicted in figure 6A 6B, both legs of mouse A, that has been injected with saline acidic solution (to simulate cancer tissue) then injected with our nanoparticles based contrast, turned bright instantly after the injection, indicating the release of the contrast from the micelles into the legs muscles, which represent clearly the switch on state of the contrast. However in the case the mouse B that has not been injected with the saline acidic solution but injected only with nanoparticles based contrast, his both legs stayed dark and no signal was observed in the MR image, indicating that these smart contrast agents remained switched off due to absence of the trigger condition (acidic condition that simulate cancer tissue). Based on these results one can say without any doubt, although is just a simulation of cancer in mouse, that this new smart contrast based on polymeric nanoparticles has the capability to remain switched off during circulation and then switch on its imaging capacity upon arrival at the target sites (tissue of interest).

To test the ability of polymeric nanoparticles to target specifically cancer tissue or cancer cells, the present inventors generated nanoparticles carrying the C595 monoclonal antibody against the human MUC1 protein, which is frequently overexpressed in cancer. For these studies the present inventors used two breast carcinoma cell lines, MCF-7 and MDA-MB-468, and replaced Gd3+ for the 1-methylpyrene (1MP) fluorescent dye (1-methylpyrene). Both cell lines expressed MUC1 mRNA (by RT-qPCR) and protein at the cell surface (by indirect immunofluorescence flow cytometry), albeit at higher levels for MDA-MB-468 cells. To test specific nanoparticle targeting, 1MP-loaded micelles bioconjugated with the C595 mAb were added to culture MCF-7 cells for 1 h. Nonconjugated 1-methylpyrene-loaded polymeric micelles the present inventors used as negative control. Using a fluorescence microscope, the present inventors observed that incubation with bioconjugated micelles conferred significantly more fluorescence to MCF-7 cells than nonconjugated micelles. Another approach was used to test the specificity of anti-MUC1 micelles. MDA-MB-468 and S17 mouse bone marrow stromal cells were cultured in a 1:5 proportion, and then incubated with anti-human MUC1 polymeric micelles for 1 hour. By doing this, the present inventors observed that MDA-MB-468 cells incorporated significantly more 1MP fluorescence than S17 cells. The specificity of anti-MUC1-conjugated micelles was also assessed by incubating MDA-MB-468 or S 17 cells with these micelles for 30 minutes and then detects cellular fluorescence by flow cytometry. After incubation with C595-bioconjugated micelles more than 8% of MDA-MB-468 cells displayed 1MP fluorescence while only 2% of S17 cells did become fluorescent. S17 or MDA-MB-468 incubation with nonconjugated micelles also resulted in approximately 2% of fluorescent cells, indicating that under these experimental conditions 2% of cells incorporate 2MP nonspecifically. Nevertheless, these experiments clearly demonstrate that bioconjugated micelles target specifically MUC1-expressing cells and not cells without antigen.

### Example 2

### DETAILED EXPERIMENT

### 1. in vitro cytotoxicity tests

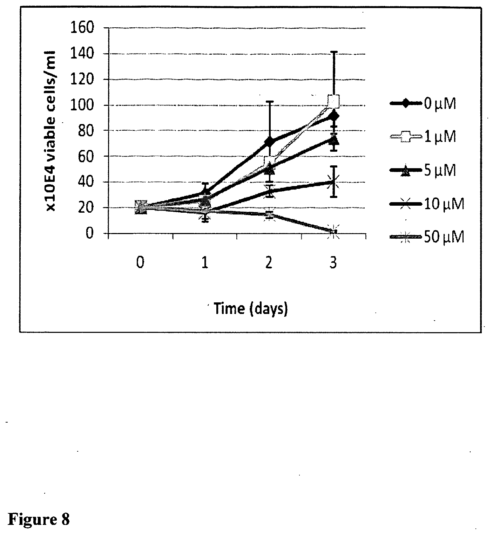

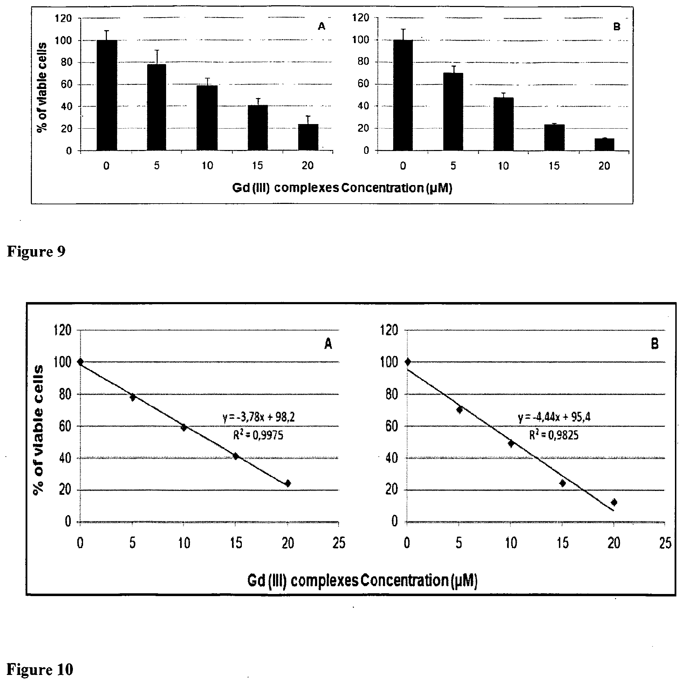

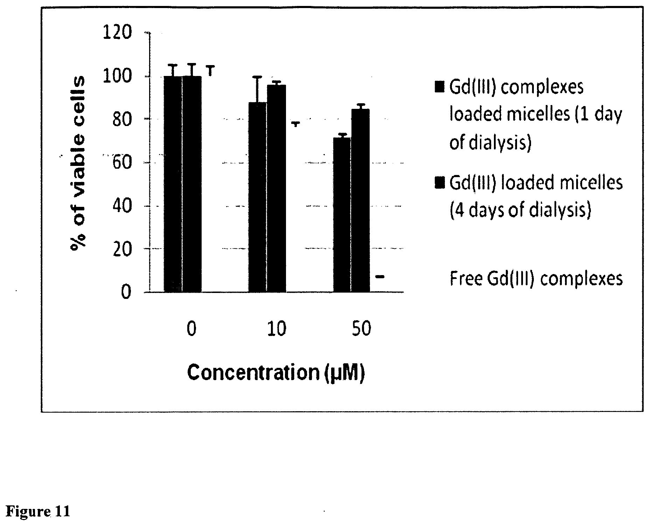

To assess the cytotoxicity of free Gd³⁺ complexes that has been synthesized by the present inventors' group (tetraaquodichloro(4,4'-ditBu-2,2'-bipyridine) gadolinium(III) chloride), the present inventors tested the effect of incremental concentrations on death of two different cell lines, Jurkat, a suspension leukemia cell line, and MCF-7, an adherent breast cancer cell line. Toxicity was represented as the number of viable cells per ml (Figure 8) or as the percentage of viable cells relatively to the control untreated group (Figure 9 and 11).

Free Gd(III) complexes were toxic for Jurkat cells at concentrations of 10 to 50 µM (Figure 8). At 10 µM, the complexes were less toxic for the cells, since even after 3 days of incubation a slight increase in cell growth was observed. In contrast, at 50 µM the complexes were very toxic, causing a progressive cell death with time.

The present inventors also evaluated the cytotoxicity of Gd(III) complexes using the MTT assay, another method measuring cell viability, on the Jurkat and MCF-7 cell lines (Figure 9 and 11). By doing so, the present inventors verified that 10 µM of Gd(III) complexes are toxic for both cell lines , significantly decreasing the number of viable cells (Figure 9). In order to estimate the concentration of Gd (III) complexes able to reduce cell viability by 50% (IC₅₀), the present inventors plotted a linear regression line for each used cell line (Figure 10). Thus, the IC₅₀ for the MCF-7 cell line was 12.8 µM of Gd(III) complexes (Figure 10-A). Fot Jurkat cells the IC₅₀ was of 10.2 µM of Gd(III) complexes (Figure 10-B). It appears thus that Jurkat cells are slightly more susceptible to Gd (III) complexes than MCF-7 cells.

To compare the cytotoxicity of free and micelle-encapsulated Gd(III) complexes and to verify if the time of dialysis influences polymeric micelle cytotoxicity, the present inventors performed again the MTT assay on Jurkat cells. The cells were treated with incremental concentrations of the free and micelle-encapsulated Gd(III) complexes.

Thus, the present inventors verified that encapsulation of Gd(III) complexes inside polymeric micelles greatly decrease their toxicity (Figure 11). The difference in cytotoxicity was most evident at 50 µM, the highest concentration tested. At this concentration, encapsulated Gd(III) complexes reduced viability to approximately 70-85% of untreated controls, while free Gd(III) complexes killed almost all cells (Figure 11). Micelles presented less toxicity for cells than free Gd(III) complexes. These experiments confirmed that 50 µM of Gd(III) complexes kill all cells, as previously verified by cell count (Figure 8).

### 2. ANALYSIS OF MUC1 GENE AND PROTEIN EXPRESSION

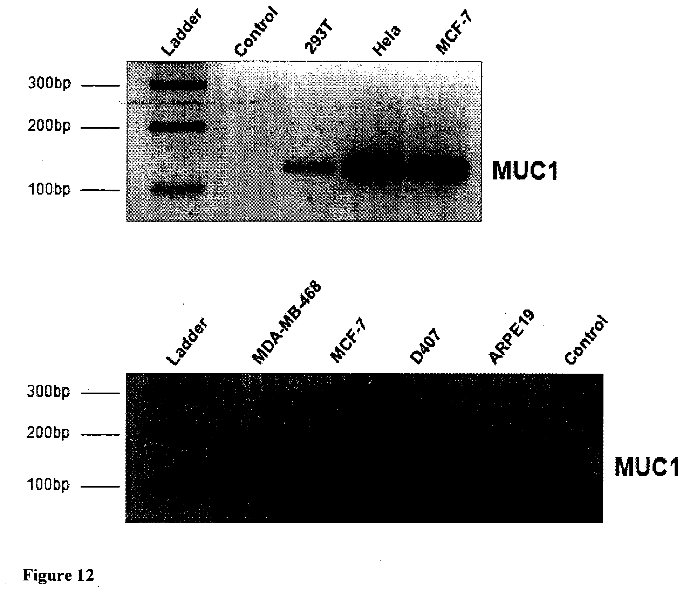

However, before initiating specific targeting tests, the present inventors verified the levels of MUC1 gene and protein expression in the cell lines used for targeting. MUC1 mRNA expression was assessed by both standard and real-time RT-PCR, whereas MUC1 protein expression was detected by Western Blotting and flow cytometry (Figures 12 to 15). (The concentration, purity and quality of RNA isolated from the different cell lines is available on Annex III.)

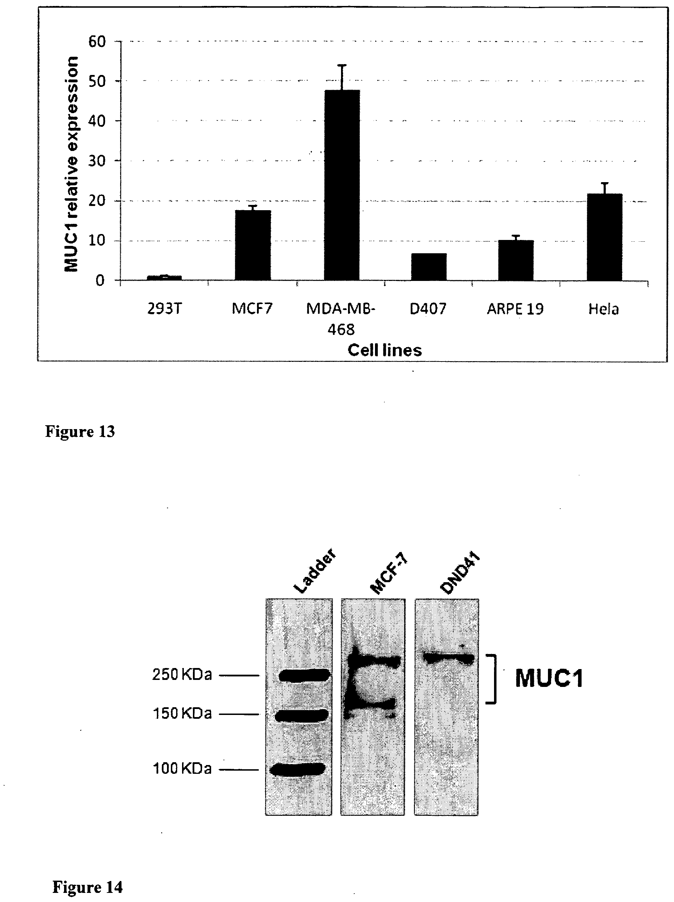

As depected in figure 13, MDA-MB-468 presented the highest levels of *MUC1* expression, when compared to the other cell lines. Hela and MCF-7 cell lines showed similarly high levels of *MUC1* expression, while the ARPE 19 and D407 retinal epithelial cell lines displayed reduced expression of *MUC1*. The human embryonal kidney 293T cell line had residual expression as compared to the other cell lines.

To confirm the RNA expression studies and detect MUC1 protein expression, the present inventors performed Western Blotting and flow cytometry. The present inventors analyzed the DND41 and MCF-7 cell lines by Western Blot.

The western blotting using the C595 monoclonal antibody revealed the expected high molecular Weight polymorphic MUC1 bands (Figure 14), demonstrating the presence of protein in MCF-7 homogenates. DND41 also expressed MUC1 protein. However, since this cell line presented just one band, only one isoform of MUC1 is present.

Also to verify the presence of MUC1 protein at the cell surface of breast cancer cell lines and to compare the expression levels between MDA-MB-468 and MCF-7 cells, the present inventors performed flow cytometry using the C595 antibody.

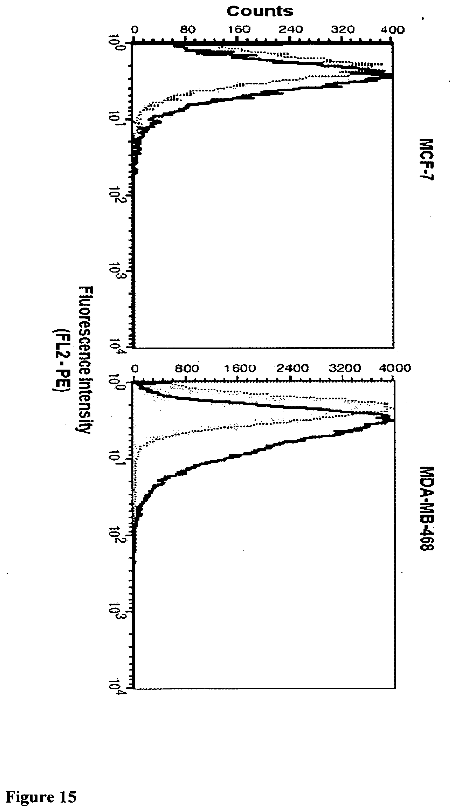

The C595 antibody revealed more fluorescence signal than an irrelevant antibody, indicating that the MUC1 protein is expressed at the cell surface of MCF-7 and MDA-MB-468 cell lines (Figure 15). However, the MUC1 cell surface expression levels were higher for MDA-MB-468 cells than for MCF-7 cells (Figure 15), thus confirming the data obtained with real-time RT-PCR.

### 3. Assessment of the targeting specificity of bioconjugated nanoparticles based smart

### contrast agent.

To confirm the capability of these polymeric micelles to target specifically MUC1-expressing cells, bioconjugated fluorophore-loaded micelles with the anti-MUC1 antibody, C595 was prepared.

In this experiment flow cytometry used to assess the ability of these bioconjuated micelles to target MDA-MB-468 cell line, which displayed the highest levels of MUC1 expression, and also S17 cell line was used as negative control. In this experiment bioconjugated and non-targeted fluorophore-loaded micelles (micelles without anti-MUC1) were incubated for different periods of time with both cell lines. Before analysis, the cells were thoroughly washed to eliminate unbound particles.

**Table 2 - Delta (Δ) values. Those values represent the faction of cells (in percentage) which showed a fluorescent intensity enhancement due to uptake or attach of 1-methylpyrene loaded nanoparticles.**

| | S17 cells | | MDA-MB-468 | |
|---|---|---|---|---|
| | **Non-targeted micelles** | **Anti-MUC1 micelles** | **Non-targeted micelles** | **Anti-MUC1 micelles** |
| 15' | 0.8% | 2.17% | 2.06% | 6.34% |
| 30' | 2.07% | 2.3% | 0.74% | 8.41% |

MDA-MB-468 cell incubation with C595 bioconjugated micelles led to a higher percentage of fluorescent cells than with non-targeted micelles (Figure 16; Table 2). In contrast, incubation of C595 bioconjugated micelles did not increase the proportion of fluorescent S17 cells, which do not express human MUC1, as compared to nontargeted micelles. It was possible to confirm the superior target capability and consequent fluorescence enhancement in MUC1-expressing MDA-MB-468 cells, as it was observed a fluorescent enhancement of 6.3-8.4% on these cells (Table 2).

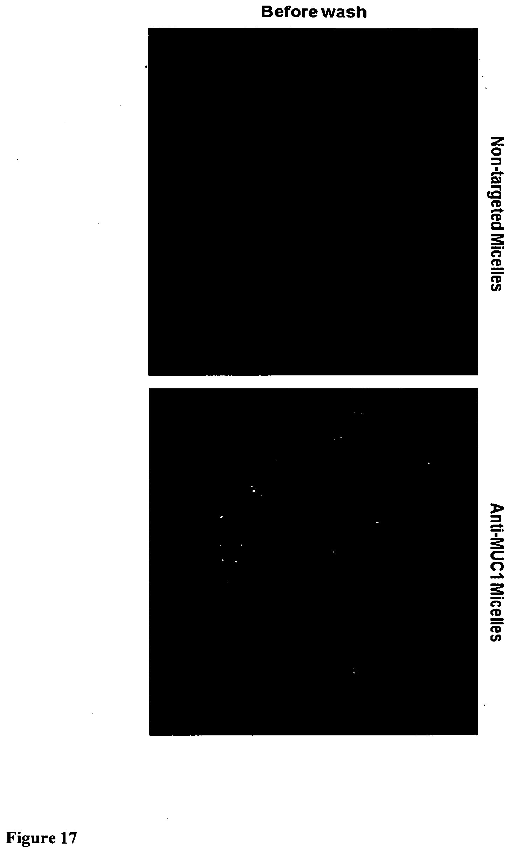

Also MCF-7 cells were incubated for 1 hour with targeted and non-targeted micelles, and then observed with an inverted fluorescence microscope. MCF-7 cells incubated with anti-MUC1 micelles presented more fluorescent staining than cells incubated with non-targeted micelles (Figure 17).

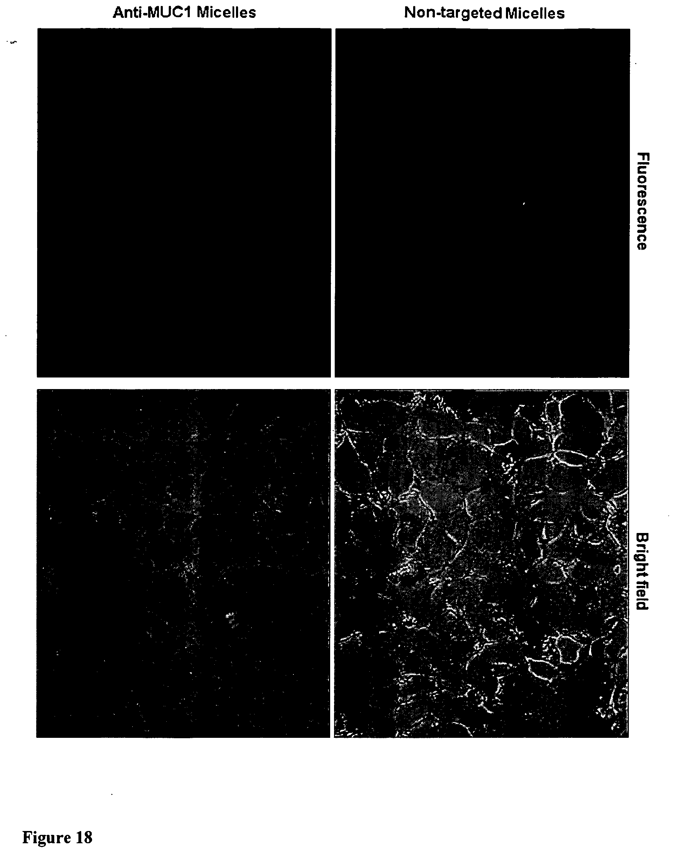

MCF-7 cells were also observed after washing with PBS, and again cells incubated with anti-MUC1 micelles presented much higher fluorescence levels than cells incubated with non-targeted micelles (Figure 18). Together, these results demonstrate the specificity of the targeted particles to MUC1-expressing cells and demonstrate that micelles release their content within cells.

Next the present inventors used another approach to test the specificity of anti-MUC1 micelles. The goal of this test was to observe if anti-human MUC1 nanoparticles were able to
specifically attach to MDA-MB-468, even when these cells were mixed in inferior number with mouse cells. Thus, MDA-MB-468 and S17 cells were cultured in a 1:5 proportion. Then the mixed culture was incubated with anti-MUC 1 polymeric micelles for 1 hour and washed several times with PBS before observation at the fluorescence microscope.

### Example 3

### EXPERIMENT SECTION

### Reagents and Equipment:

All reagents and solvents for synthesis were reagent grade and were used without further purification, unless stated otherwise. PEG 2'000 monomethyl ether mesylate and 1-methylpyrene (for fluorescence grade) were purchased from Fluka. Potassium O-ethyl xanthate was purchased from Sigma-Aldrich. Trimethylsilyl methacrylate was purchased from Sigma-Aldrich and distilled at low pressure in a Büchi Glass Oven B-585 micro distiller before use. Azobisisobutyronitrile (AIBN) was purchased from Fluka, crystallized from methanol and dried under vacuum at room temperature. Anhydrous sodium sulphate and hydrochloric acid were purchased from Panreac. Magnetic beads were purchased from Chemicell, Germany. The anti-*Escherichia coli* antibody was purchased from AbD Serotec and the anti-MUC 1 antibody was purchased from Santa Cruz Biotechnology. Methoxypolyethylene glycol Nhydroxysuccinimide ester was purchased from Sigma-Aldrich. Methanol for GPC was HPLC grade, from Merck. For dialysis and GPC milliQ water was used. The dialysis cassettes were Pierce Biotech, 10 kDa MWCO. Centrifugations were carried out in a Beckman Coulter Avanti J-25 I centrifuge. Triple detection GPC (SEC3) analysis was performed in a modular system: degasser, HPLC pump (K-1001) and RI detector (K-2300) are from Knauer; viscometer and RALLS are from Viscotek (Trisec Dual Detector Model 270), using two PL aquagel-OH mixed 8 µm 300 x 7.5 mm columns and 20% MeOH / 80% 0,1 M NaNO₃ as eluent. ¹H NMR analysis was performed in a Bruker ARX 400 (400 MHz). Steady-state fluorescence spectra were recorded on a SPEX Fluoromax-3 spectrofluorimeter (Jobin Yvon - Horiba, France). All emission spectra were carried out in 1-cm quartz cuvettes, using *λ*_{exc}=334 nm, collecting the emission from 360 to 510 nm (increment of 1 nm), using 1-nm slit widths in excitation and emission (wavelength resolution of 1 nm), and corrected for nonlinear instrument response. To assess in vitro cytotoxicity of loaded and unloaded gadolinium in the polymeric micelles was used a 3-(4, 5-dimethylthiazol-2-yl)-2, 5-diphenyl tetrazolium bromide (MTT) assay, which were purchased from AppliChem. To measure the absorbance of the formazan product was used a Tecan Infinite M200 monochromator-based multi-function microplate reader (test wavelength of 570 nm and a reference wavelength of 630 nm). Fluorescence imaging was performed with an inverted Leica DM IL microscope and a 40x objective lens. The photos were captured using a digital CCD camera (Leica DC 500). The photos were processed using Adobe Photoshop CS2 software (AdobeSystems).

### 1- Polymer synthesis

### 1a) Macro-CTA (Chain Transfer Agent) synthesis.

0.8 g (5 eq) potassium O-ethyl xanthate were suspended on 15 mL of DCM (dichloromethane) in a two-necked round-bottomed flask and a solution of 2 g of PEG 2'000 monomethyl ether mesylate, in 10 mL of DCM added dropwise, under stirring, while heating. The mixture was refluxed for 48 h, with one addition of an equal amount of solid xanthate with a spatula, after 24 h. The mixture, initially and after the addition yellow, becomes paler. The flask content was filtered to a separatory funnel and washed three times with equal volume of water. The organic phase was then dried over anhydrous sodium sulphate and concentrated in a rotary evaporator. The polymer was thus precipitated by addition of diethyl ether, recovered by filtration and dried over night, under vacuum, at 40 °C, affording 0.92 g (46%) of a white solid. ¹H NMR (CDCl₃) 1.402 (t, **CH₃**CH₂O-C=S), 3.361 (s, **CH₃**O-(CH₂CH₂O)*ₙ*), 3.626 (m, (**CH₂CH₂**O)*ₙ*), 4.630 (q, CH₃**CH₂**O-C=S).

### 1b) Diblock co-polymer synthesis.

0.8 g of the macro-CTA were placed in a Schlenk tube, previously charged with nitrogen, and dissolved in 10 mL of THF. 3.5 mL (45 eq) of trimethylsilyl methacrylate and 0.030 g (1 mol % of the monomer) of AIBN were added. After three freeze-pump-thaw cycles, the mixture was incubated in an oil bath at 60 °C, under stirring, for 24 h. The flask was then cooled down to room temperature, 10 mL of THF added to dilute the mixture, which had become very viscous, and its content transferred to a round bottomed flask. After evaporation of the solvent, the white residue was dissolved in methanol and the polymer precipitated by the addition of water. The polymer was separated from the supernatant by centrifugation at 15,344xg during 30 min, followed by decantation. This procedure was repeated once more, affording a white solid that was vacuum dried, at 60 °C, overnight, yielding 1.47 g (40%). ¹H NMR (DMSO) 0.056 (s, (**CH₃**)₃Si), 1.086 (s, **CH₃**C-C=O), 1.177 (s, **CH₃**C-C=O), 1.878 (s, **CH₂**C-C=O), 1.992 (s, **CH₂**C-C=O), 3.356 (s, **CH₃**O-(CH₂CH₂O)*ₙ*), 3.639 (s, (**CH₂CH₂**O)*ₙ*), 4.660 (q, CH₃**CH₂**O-C=S). DP*ₙ* (NMR) = 45. GPC analysis (PSSNa standards) reveals a monomodal molecular weight distribution, from which a value of *Mₙ* = 9 kDa was estimated.

### 2- PREPARATION OF Gd(III) COMPLEX SOLUTION

Hydrophobic Gd⁺³ complexes (tetra-aquodichloro (4,4'-ditBu-2,2'-bipyridine) gadolinium (III) chloride) were synthesized. The contrast agent was synthesized based on a well-described method to produce hydrophobic Gd(III) complexes with ligands such as bipyridine (Bechara, et al., 2009). The resultant Gd(III) complex has a molar mass (M) of 604.067 g.mol⁻¹. To get a 50 mM stock solution, 5.1 mg of Gd(III) complex were dissolved in 169 µL of ethanol (96%)(AGA). The solution was kept at -20°C.

### 3- POLYMERIC MICELLE PREPARATION

### 3a- Preparation of Gd(III) complex-loaded polymeric micelles

Loaded polymeric micelles with Gd(III) complexes were obtained as follows: 30 mg of poly(ethylene glycol-*b*-trimethylsilyl methycrylate) and 0.5 mg of Gd(III) complexes were dissolved in 0.3 mL of **N,N-Dimethylformamide** (DMF) (BDH Prolabo^{®}). The mixture was stirred for 3 hours at room temperature. Subsequently, 0.7 mL of pure Milli-Q water was added at a rate of one drop every 10 s to induce micellization. The resulting solution was then placed in a dialysis cassette (Thermo Scientific) with a 10 kDa cut-off and dialyzed against Milli-Q water for 1 day at room temperature. Then 0.5 mL from the solution in the dialysis cassette were collected and the remaining was dialyzed for 3 days at room temperature. The milli-Q water was replaced twice a day.

### 3b- Preparation of fluorophore-loaded polymeric micelles

Polymeric micelles loaded with the 1-methylpyrene fluorophore were prepared by adding 30 mg of poly(ethylene glycol-*b*-trimethylsilyl methycrylate) to 0.5 mg of 1-methylpyrene (Fluka) and dissolved in 0.4 mL of DMF. The mixture was stirred for 3 hours at room temperature. Subsequently, 1 ml of Milli-Q water was added at a rate of one drop every 10 s to induce micellization. The resulting solution was then placed in a dialysis cassette with a 10 kDa cut-off and dialyzed against Milli-Q water for 2 days at room temperature. The milli-Q water was replaced twice a day.

### 4- BIOCONJUGATED MICELLE PREPARATION

### 4a- Bioconjugation of polyethylene glycol with anti-MUC 1

Bioconjugation of polyethylene glycol with mouse monoclonal antibody against the human MUC1 protein (C595; Santa Cruz Biotechnology) was completed as follows: 3 mg of methoxypolyethylene glycol N-hydroxysuccinimide ester (Sigma-Aldrich) was added to a solution containing 3 µg of C595 (30 µL) in 0.1 M bicarbonate buffer (500 µL, pH=8.3). The mixture was gently stirred for 5 days in slow tilt rotation at 4°C.

### 4b- Preparation of fluorophore-loaded bioconjugated micelles

Loaded bioconjugated micelles were prepared by adding 30 mg of poly(ethylene glycol-*b-*trimethylsilyl methycrylate) with 0.5 mg of 1-methylpyrene and dissolved in 0.4 mL of DMF. The mixture was stirred for 3 hours at room temperature. Subsequently, 0.5 mL PEG-Anti-MUC1 aqueous solution was added at a rate of one drop every 10 s and, immediately after, 0.5 mL of cold Milli-Q water was added at the same rate to induce micellization. The resulting solution was then placed in a dialysis cassette with a 10 kDa cut-off and dialyzed against cold Milli-Q water for 1 day at 4°C. The Milli-Q water was replaced once.

### 5- Detection of contrast agent "on" and "off" states by using clinical mri

To image Gd(III) complexes released from Gd(III) complex-loaded micelles a 1.5 Tesla MRI (**Signa 1.5T SYS#GEMSOW,** GE Healthcare) was used. The same volume of a solution with Gd(III) complexes loaded micelles in milliQ water was dispensed in two different microfuge tubes. Next to one of these microfuge tubes a few drops of HCL (37%) (Merck) were added. Both tubes were positioned above a **MRI phantom** for **calibration.** MR images were subsequently acquired with a Fast Spoiled Gradient Echo sequence (TR/TE= 150/4.2 milliseconds; Flip angle, 90°; slice thickness, 4 mm; field of view, 24x18 cm; matrix, 256x256).

### 6- Measurement of gd(iii) complexes and fluorophore concentrations

### 6a-methylpyrene calibration curve

From a stock solution of 2.17x10⁻⁵ M of 1-methylpyrene in tetrahydrofuran (THF, HPLC grade, Ridel-de-Haën), five standards of concentrations 1.63x10⁻⁵, 1.09x10⁻⁵, 8.68x10⁻⁶, 5.43x10⁻⁶ and 1.09x10⁻⁶ M were prepared. The standard absorbances were measured (Varian Cary 50 UV-Vis spectrophotometer) at 344 nm and the calibration curve obtained by fitting the absorbance with concentration data. A value of 3.01x10⁴ dm³.mol⁻¹.cm⁻¹ for the molar absorption coefficient was found. Given the calibration curve, the absorbance from two different samples, one of bioconjugated 1-methilpyrene loaded micelles and other from non-target micelles was measured. For measurements THF was added to 30 µl of a sample with unknown concentration until reaching 3 mL in the cuvette, obtaining a 100x dilution. For each sample the absorbance was measured in triplicate.

### 6b-Gd(III) complex calibration curve

A stock solution of Gd(III) complex in ethanol (96%, AGA) 6.62x10⁻⁵ M was diluted to obtain standards with the following concentrations: 3.31x10⁻⁵, 1.66x10⁻⁵, 3.31x10⁻⁶, 1.66x10⁻⁶ and 3.31x10⁻⁷ M, whose absorbances were recorded at 282 nm. The molar absorption coefficient was 1,26x10⁴ dm³.mol⁻¹.cm⁻¹.

### 7- Cell lines and culture conditions

### 7a-Cell Lines

The Jurkat (human T-cell leukemia), MCF-7 and MDA-MB-468 (human breast adenocarcinoma) cells lines were kindly supplied by Dr. João Barata (IMM, Lisbon), Dr. Raquel Seruca and Dr. Joana Paredes (IPATIMUP, Porto) respectively. The S17 (mouse bone marrow stromal) cell line was provided by Dr. Leonor Parreira (IMM, Lisbon). HeLa (human cervical cancer), and 293T (human Embryonic Kidney) cell lines were provided by Dr. Guilherme Ferreira (IBB/CBME, Faro); DND41 (human T-cell leukemia) cell line by Dr. Hind Medyouf (Terry Fox Laboratory, Vancouver) and human retinal pigment epithelium cell lines, ARPE19 and D407, by Dr. Gabriela Silva (IBB/CBME, Faro).

### 7c- Cell Culture

**The** MCF-7, MDA-MB-468, HeLa, 293T; ARPE19; D407 and S17 adherent cell lines were maintained in DMEM medium (LONZA) supplemented with 10% fetal bovine serum (FBS) (PAA), glutamine (200 mM), penicillin (10000 units/mL) and streptomycin (10000 µg/mL) from LONZA. All cell culture incubations were performed at 37°C under humidified atmosphere of 5% CO₂. The Jurkat and DND41 suspension cell lines were maintained in RPMI 1640 medium (LONZA) supplemented as above. Cells were thawed at 37°C and pipetted into 10 mL of prewarmed medium. The cells were centrifuged at 300 g for 5 minutes to remove dimethylsulfoxide (DMSO) (Merck). Then, the supernatant was discarded and the pellet resuspended in 2 mL of fresh medium. The cells were placed into T25 flasks (Nunc) and 8 mL of medium was added for a final volume of 10 mL. The medium was changed every 2 days. To count adherent cells, these were first detached by removing the medium, followed by washing with phosphate-buffered saline (PBS) (LONZA) and addition of 1 mL of 0.25% trypsin (LONZA) per 59 cm². Then, the cells were allowed to detach for 1 to 5 minutes (depending on cell line) at 37°C, before resuspension in DMEM medium. To dissociate cell aggregates, both suspension and adherent cells were gently pipetted. Then, 30 µL of cell suspension were placed into a microfuge tube together with 30 µL of 0.4% Trypan Blue (Sigma-Aldrich) and mixed. The hemocytometer Neubauer chamber (Hausser Scientific) was filled with the cell suspension and uncolored viable cells were counted on an inverted light microscope (Leica DM IL). The cells were counted in four 1 mm² squares. The equation to calculate the cell number per cubic millimeter is the following: Number of cells counted per square millimeter × dilution used × 10.

### 8- IN VITRO CYTOTOXICITY TESTS

### 8a- Cell counting to assess Gd(III) complex cytotoxicity

Jurkat cells were seeded on 6-well culture plates (BD Falcon) (2 mL in each well with a concentration of 2x10⁵ cells/mL). Different concentrations (1 µM; 5 µM; 10 µM; 50 µM) of Gd(III) complex solution, diluted in 50 µl RPMI-1640, were added to the cells. Each test was performed in duplicate. Untreated cells served as control groups. Cells were incubated for 3 days, and each day cells were collected for counting.

### 8b- MTT assay to assess Gd(III) complex concentration cytotoxicity

Two different cell lines were used to assess Gd(III) cytotoxicity: Jurkat, and MCF-7. Jurkat cells were seeded on two 96-well culture plates (BD Falcon) (50 µl in each well with a concentration of 4x10⁵ cells/mL). Different concentrations (5 µM, 10 µM, 15 µM and 20 µM) of Gd(III) complex solution, diluted in 50 µl of RPMI-1640, were used. Each test was repeated eight times. As a negative control, cells were treated with ethanol (96%) (the same amount of ethanol present in the cells treated with a highest concentration of Gd(III) complex). Incubation was carried out for 3 days. In this experiment the toxicity of Gd(III) complex was evaluated by the 3-(4, 5-dimethylthiazol-2-yl)-2, 5-diphenyl tetrazolium bromide (MTT) assay. At the end of each incubation time 10 µl of 5 mg/mL MTT solution (AppliChem) were added to each well followed by further incubation for 4h. Then, 100 µl of 0.04 N HCL in isopropanol (BDH Prolabo) were added to the wells and formazan crystals were allowed to solubilize for 1 hour at room temperature. The formazan concentration was quantified using a spectrophotometer (Tecan Infinite M200 monochromator-based multi-function microplate reader) by measuring the absorbance at 570 nm and 630 nm. The final optical density (OD) obtained from formazan formation was calculated with the following formula: OD=L₍₅₇₀ₙₘ₎-L₍₆₃₀ₙₘ₎. The toxicity was assessed by calculating the % of cell survival in relation to the control untreated group. The procedure described above was repeated for the MCF-7 cell line, after being seeded at 5 × 10³ cells per each well and allowed to attach for 24h.

### 8c- MTT assay to assess Gd(III) complex-loaded polymeric micelle cytoxicity

To assess Gd(III) complex-loaded polymeric micelle cytoxicity and to compare it with the toxicity from free Gd(III) complex, Jurkat cells were seeded on a 96-well culture plate (50 µl in each well with a concentration of 4x10⁵ cells/mL). Different concentrations of Gd(III) complex and Gd(III) complex-loaded polymeric micelles dialyzed for 24 hours or for 96 hours (at concentrations of 10 µM or 50 µM) diluted in 50 µl of RPMI-1640 were added to the cells. Each test was performed in quadruplicate. Untreated cells, or cells treated with solvent (ethanol or Milli-Q water) served as control groups. Cells were incubated for 72h. The cytotoxicity of Gd(III) complex and Gd(III) complex-loaded polymeric micelles was evaluated by the MTT assay following the procedure mentioned on 8.2.

### 9- ANALYSIS OF MUC1 EXPRESSION

### 9a- SDS-PAGE and Western Blotting

### i. Cell lysis in RIPA buffer

DND41 and trypsinized MCF-7 cell lines were washed twice in 1 mL of cold PBS. The supernatant was discarded and PBS removed without disturbing the cell pellet. Protein extracts were obtained by lysing cells in ice-cold RIPA buffer [10 mM Tris, pH 7.4; 150 mM NaCl; 1 mM EDTA; 1% Triton (100x); 0.5% Na deoxycholate; 0.1% SDS] with freshly added protease inhibitors (10 µg/mL aprotinin; 10 µg/mL leupeptin and 1mM PMSF). 100 µL of RIPA buffer were added for each 10 million cells. After homogenization, the tubes were incubated on ice for 10 minutes. The cell debris was removed by centrifugation at 14000 rpm for 10 and transferring the cleared cell lysate to a new microfuge tube. Samples were stored at -80°C. For electrophoresis, 60 µL of each lysate were mixed with 60 µL of 2 × SDS sample buffer (62.5 mM Tris, pH 6.8; 20% glycerol; 2% SDS; 5% β-mercaptoethanol; bromophenol blue). Subsequently the proteins were denaturated at 95°C for 4 minutes.

### ii. SDS-PAGE

An 8% separating polyacrylamide gel was prepared using 30% acrylamide/bisacrylamide (37.5:1) (ProtoGel); 1M Tris (pH 8.8); 10% SDS; 10% ammonium persulphate (Applichem); N,N,N'N'-tetramethylethylenediamine (TEMED) Electran (BDH); and deionized water. A 4.5% stacking gel was prepared with the same reagents with exception of buffer, which was 5.5 M Tris, pH 6.8. The polymerized gels were prepared on a mini-Protean (Bio-Rad) apparatus, following the manufacturer's instructions. Tris-glycine electrophoresis buffer (25mM Tris, pH 8.3; 192 mM glycine; 0.1% SDS) was added until SDS-PAGE submersion. Equal amounts (20 µL) of protein from MCF-7 and DND 41 cell lines were loaded onto SDS-PAGE; and 5 µL of pre-stained high molecular weight protein markers (Precision Plus Protein Standards, Bio-Rad) were used as molecular weight standards. Gels were ran at 200 V constant voltage for approximately 45 minutes.

### iii. Western Blotting

Migrated proteins were electroblotted onto a 0.2 µm nitrocellulose membrane (Schleicher & Schuell). To this end, the gel was sandwiched between a pre-wet nitrocellulose membrane, six pieces of filter paper (Whatman 3MM) and fiber pads. The cassette was placed in the modular electrode assembly inside the buffer tank and filled with cooled transfer buffer (25mM Tris; 192mM glycine; 20% methanol). Electroblotting was carried out for 1 hour at 100 V constant voltage. Membrane blocking for 30 minutes and all antibody incubations were done at room temperature with 5% non-fat dried milk dissolved in 10 mL of PBS/0.1% Twe 20 (BDH Prolabo) (PBST). For protein detection, membranes were incubated sequentially with C595 MUC1 monoclonal antibody (diluted 1/500) for 1 hour; and horseradish peroxidase (HRP)-conjugated goat anti-mouse (AbD SEROTEC), (diluted 1/1000) during 45 minutes. Excess antibodies were washed out 3 times during 10 minutes with PBST. All antibody incubations and washes were performed at room temperature with slow tilt oscillation. HRP detection was performed using the chemiluminescent detection kit, SuperSignal west Pico Chemiluminescent Substrate (Pierce) following the manufacturer's instructions. Chemiluminescence was revealed by exposing the nitrocellulose membrane to a radiographic film (Amersham Hyperfilm - GE healthcare Life Sciences). For antibody dehybridization, the nitrocellulose membrane was incubated during 30 minutes at room temperature with a solution of 100 mL of distilled water with 570 µL of 100% acetic acid, and submitted to 3 washes of 10 minutes with PBST. Then, it was incubated with α-tubulin monoclonal antibody (Sigma-Aldrich) (diluted 1/10000) for 1 hour. Signal detection was done as described above for MUC1 antibody.

### b. RT-PCR

### i. RNA Extraction

Total RNA was extracted from MDA-MB-468, MCF-7, Hela, 293T, ARPE19 and D407 cell lines. The cells were cultured on 60 mm culture dishes and allowed to grow until sub-confluence. The medium was removed, culture dishes were washed with PBS and 1 mL of TRIZOL reagent (Invitrogen) was added directly to the cells. The cells were lyzed by repeated pipetting, and allowed to incubate at room temperature for 5 minutes. Subsequently, 0.2 mL of chloroform (BDH Prolabo) was added to the cells, the tubes were closed, shaked vigorously by hand for 15 seconds and allowed to incubate at room temperature for 10 minutes. Next, the tubes were centrifuged at 12000 g, for 10 minutes at 4°C and the aqueous phase transferred to a new clean 2 mL microfuge tube. To precipitate RNA, 0.5 mL of isopropanol (BDH Prolabo^{®}) was added to the aqueous phase and mixed. The samples were incubated at room temperature for 10 minutes. Then, the samples were centrifuged at 12000 g, for 10 minutes at 4°C. The supernatant was discarded; and 1 mL of 75% RNase-free ethanol was added, mixed by vortexing and centrifuged at 7500 g for 5 minutes at 4°C. The supernatant was discarded and the samples were allowed to dry by vacuum for 10-15 minutes. Depending on the size of the obtained visible RNA pellet, 20 to 50 µL of RNase-free water was added to dissolve the pellet, and incubated for 10 minutes at 60°C. The concentration and purity of isolated RNA was measured by NanoDrop 2000c Spectrophotometer (Thermo Scientific), and its quality assessed by 1% agarose (LONZA) gel migration in TAE buffer (40 mM Tris-Acetate; 0.5 mM EDTA, pH 8.5). The RNA samples were stored at -80°C.

### ii. DNase treatment

Treatment of RNA with DNase I, RNase-free (Fermentas, #EN0521) was performed to obtain DNA-free RNA for RT-PCR. Thus, 1 µg of RNA in 9 µL of DEPC-treated water was added to 1 µL of reaction buffer (10x) [100 mM Tris-HCl (pH 7.5 at 25°C), 25 mM MgCl₂, 1 mM CaCl₂] and 1 µL of DNase enzyme (1 U/ µL). The reagents were mixed, centrifuged to collect drops, incubated at 37°C for 30 minutes and briefly centrifuged again. Then 1 µL of 25 mM EDTA was added, and the solution incubated at 65°C for 10 minutes to inactivate the enzyme. After incubation the samples were used as templates for reverse transcription.

### iii. Reverse transcriptase reaction

Reverse transcription was conducted using the RevertAid First Strand cDNA Synthesis Kit (Fermentas, #K1621) according to the manufacturer's intructions. To the previously obtained 11 µL of DNase-treated RNA, 1 µL of oligo (dT)₁₈ primer (0.5 µg/ µL) was added. The sample was mixed, briefly centrifuged to collect drops and incubated at 70°C for 5 minutes. Then, the mix was cooled on ice and shortly centrifuged. Next, 4 µL of reaction buffer (5x) [250 mM Tris-HCL (pH 8.3), 250 mM KCl, 20 mM MgCl₂, 50 mM DTT], 1 µL of RiboLock Ribonuclease Inhibitor (20U/ µL), and 2 µL of dNTP mix (10 mM) were added. The mix was briefly centrifuged and incubated at 37°C for 5 minutes. Afterwards, 1 µL of RevertAid M-MuLV Reverse Transcriptase (200 U/ µL) was added to the mixture, and this was incubated at 42°C for 60 minutes. To stop the reaction, the mix was heated at 70°C for 10 minutes. Finally, the sample was cooled down on ice and stored at -20°C.

### iv. Polymerase Chain Reaction (PCR)

For PCR amplification a mix was prepared composed of: 2 µL of DNA sample, 5 µL Go Taq Flexi reaction buffer (5x) (without MgCl₂), 1.5 µL of MgCl₂ (25 mM); 0.5 µL of dNTP (10 mM), 1 µL of forward primer and 1 µL of the reverse primer (12.5 µM), 0.1 µL of GoTaq DNA polymerase (5 U/µL) and sterile deionized water until a final volume of 25 µL. All the reagents mentioned above were from Promega with the exception of dNTPs (Fermentas). The PCR reaction was performed in a thermal cycler (MyCycler, Bio-Rad) with the following program: an initiation step of 95°C for 2 minutes, followed by 30 cycles of denaturation at 95°C for 1 minute, annealing at 55°C for 1 minute and elongation at 72°C for 30 seconds. A final elongation step of 72°C for 5 minutes was also performed. The *MUC1* transcript was amplified using the following primer sequences, designed using Primer3 software and synthesized by Sigma-Aldrich Forward, 5'-GTG CCC CCT AGC AGT ACC G-3'; and Reverse, 5'-GAC GTG CCC CTA CAA GTT GG-3'. After PCR amplification, 10 µL of each PCR reaction product and 5 µL of 100 pb ladder (Bioron) were loaded into a 2% agarose gel and electrophoresed in TAE buffer. The PCR products were visualized with a UV light transilluminator attached to a digital camera (Gene FLASH).

### c. Real-time PCR

For real-time PCR, the present inventors used the iQ SYBR⁻Green Supermix kit (Bio-Rad) following the manufacturer's instructions. The cDNA samples obtained as described at 7.2.3., were used at a 1/20 dilution. A master mix was prepared for each pair of primers with 9.7 µL of H₂O, 0.4 µL of forward primer, 0.4 µL of reverse primer (both at 12.5 µM) and 12.5 µL of Sybr Green Master Mix (2x). For *MUC1* detection the present inventors used the primers described on 7.2.4. For *ACTB* detection, used as a reference gene, the present inventors used the following primers: Forward, 5'-AGG CCA ACC GCG AGA AGA TGA C-3' and Reverse, 5'-AGG TCC AGA CGC AGG ATG GCA T-3' (Villablanca, et al., 2008), synthesized by Sigma-Aldrich. The master mix was distributed on 96-Well ABgene PCR plates (Thermo Scientific, AB-0700), 23 µL per well, together with 2 µL of sample cDNA (each sample in triplicate). Water was used as negative control. The PCR microplate was covered with the adhesive PCR film (Thermo Scientific, AB-0558). The real-time PCR was performed on a CFX96 Real-Time PCR Detection System (Bio-Rad), the reaction program used was the same described at 9.2.4. Relative mRNA expression levels were calculated through the 2^{-ΔΔCt} method (Livak & Schmittgen, 2001).

### d. Flow Cytometry

Flow cytometry was performed on trypsinized MCF-7 and MDA-MB-468 cells, resuspended in cold PBS. 5x10⁵ cells of each cell line were dispersed by gently aspirating with a pipette, placed in each of three 5 ml FACS tube (BD Falcon™) and resuspended in 1mL of cold FACS buffer [3% Calf Serum (CS) (PAA), 10 mM NaN₃ in PBS]. Two of the three FACS tubes of each cell line were centrifuged at 300 g for 5 minutes. The supernatant was discarded, the cell pellet of one FACS tube was incubated with 50 µL of C595 antibody (1:100 in FACS buffer) and the other tube with α-tubulin antibody (1:500 in FACS buffer). The incubation was carried out for 1 hour. Then, the cells were washed twice with 1 mL of FACS buffer and then incubated with **phycoerythrin-conjugated goat anti-mouse immunoglobulins** (GAM-PEs) (Biolegend) diluted 1:100 in FACS buffer, in the dark for 30 minutes. As a control group, 5x10⁵ cells were incubated with GAM-PE only. The cells were washed as above and resuspended in 1mL of 10 mM NaN₃ in PBS. All the mentioned steps were performed at 4°C. Finally the cells were analyzed using the BD FACSCalibur (BD Biosciences) equipped with the Cell Quest software package (BD Biosciences).

### 10- ASSESSMENT OF SPECIFIC TARGETING OF BIOCONJUGATED NANOPARTICLES

### e. Flow Cytometry

MDA-MB-468 and S17 cells were seeded on 12-well culture plates (6x10⁴ cells/well in 600 µL of DMEM) and allowed to attach for 24 hours. Then, 200 µL of anti-MUC1-targeted and non-targeted micelles with encapsulated fluorophore were added to each cell line. Cells were incubated with targeted micelles or non-targeted micelles for 15 or 30 minutes at 37°C. Untreated cells were used as negative control. After incubation, the medium was discarded and the cells were washed 3 times with 1 mL cold PBS. Subsequently, the cells were detached with 0.25% trypsin and resuspended in 1 mL cold PBS. These samples were filtered through a cell-strainer cap (BD Falcon) to 5 mL FACS tubes. The cells were centrifuged at 300 g for 5 minutes and resuspended in 1 mL of cold PBS. All the mentioned steps were performed at 4°C. Finally the cells were analyzed using the BD FACSAria II Multicolor cell sorter (BD Biosciences) and data were examined by Cell Quest software package (BD Biosciences).

### f. Fluorescence microscopy

### i. Without cell fixation

MCF-7 cells were seeded in 96-well culture plates (50 µl in each well with a concentration of 5x10³ cells per well) and allowed to attach for 24 h at 37°C. Micelles bioconjugated with C595 (25 µl) and loaded with 1-methylpyrene were added to the cells and incubated for 1 hour at 37°C. As a control, the present inventors used unconjugated 1-methylpyrene-loaded polymeric micelles. Excess micelles were removed by three times washing with PBS. Microscopy observations and photography of cells before and after micelle washing were performed with an inverted fluorescent microscope (Leica DM IL). Photographs were obtained with a digital CCD camera (Evolution MP-5.1, Media Cybernetics). The photos were processed using Adobe Photoshop CS2 software (AdobeSystems).

### ii. With cell fixation

Sterile 13 mm glass coverslips (VWR) were placed inside two wells of a 12-well culture plate. On one of those wells MDA-MB-468 and S17 cells were seeded with the proportion of 1 to 5; on the other well MFC-7 and S 17 cells were seeded with the same proportion. To both wells 600 µL of DMEM medium were added to make a final concentration of 6x10⁴ cells per well. The cells were allowed to attach for 24 hours at 37°C. Then, 200 µL of anti-MUC1 fluorophore-loaded micelles were added to the cells and incubated for 1h at 37°C. Then, the cells were washed 3 times with 1 mL of PBS and fixed with 600 µL of paraformaldehyde (4%) (Sigma-Aldrich) for 30 minutes. The cells were washed once with 1 mL of PBS, and each coverslip was careful collected from the wells with forceps and layed onto a microscope slide (Menzel-Glaser) and covered with a Mowiol mounting (Hoechst). The glass slides were kept cold overnight. Microscopic observations and photography of fixed cells were performed with Axio Observer Z2 Fluorescence microscope (Zeiss). Photographs were obtained with a digital AxioCam (Zeiss) and were processed using AxioVision is the software (Zeiss).

### REFERENCES

1) Weissleder, R., Mahmood, U. (2001) Molecular imaging. Radiology 219, 316-333
2) Massoud, T. F., Gambhir, S. S. (2003) Molecular imaging in living subjects: seeing fundamental biological processes in a new light. Gene Dev. 17, 545-580.
3) Weissleder, R., Lee, A. S., Fischman, A. J., Reimer, P., Shen, T., Wilkinson, R., Callahan, R. Brady, T. J. (1991) Polyclonal human immunoglobulin G labeled with polymeric iron oxide antibody MR imaging. Radiology 181, 245-249.
4) Krieg, F. M., Andres, R. Y., Winterhalter,K. H. (1995) Superparamagnetically labeled a. neutrophils as potential abscess-specific contrast agent for MRI. Magn. Reson. Imaging 13, 393-400
5) Kang, H. W., Josephson, L., Petrovsky, A., Weissleder, R., Bogdanov, Jr., A. (2002) Magnetic resonance imaging of inducible e-selectin expression in human endothelial cell culture. Bioconj. Chem. 13, 122-127
6) Zhao, M., Beauregard, D. A., Loizou, L., Davletov, B., Brindle, K. M. (2001)Noninvasive detection of apoptosis using magnetic resonance imaging and a targeted contrast agent. Nat. Med. 7, 1241-1244
7) Ho¨gemann, D., Josephson, L., Weissleder, R., Basilion, J. P. (2000) Improvement of MRI probes to allow efficient detection of gene expression. Bioconj. Chem. 11, 941-946
8) Wadghiri, Y. Z., Sigurdsson, E. M., Sadowski, M., Elliott, J. I., Li, Y., Scholtzova, H., Tang, C. Y., Aguinaldo, G., Pappolla, M., Duff, K., Wisniewski, T., Turnbull, D. H. (2003) Detection of Alzheimers amyloid in transgenic mice using magnetic resonance microimaging. Magn. Reson. Med. 50, 293-302
9) Jun, Y.-W., Huh, Y.-M., Choi, J.-S., Lee, J.-H., Song, H.-T., Kim, S. J., Yoon, S., Kim, K.-S. Shin, J.-S., Suh, J.-S., Cheon, J. (2005) Nanoscale size effect of magnetic nanocrystals and their utilization for cancer diagnosis via magnetic resonance imaging. J. Am. Chem. Soc. 127, 5732-5733
10) Gerweck LE and Seetharaman K (1996) Cancer Res 56:1194
11) Zhang S,Wu K and Sherry AD (1999) Angew Chem Int Ed 38:3192
12) Moats RA, Fraser SE and Meade TJ (1997) Angew Chem Int Ed 36:726
13) Li W, Fraser SE and Meade TJ (1999) J Am Chem Soc 121:1413
14) Grynkiewicz G, Poenie M and Tsien RY (1985) J Biol Chem 260:3440.
15) W. Hamley. Nanoshells and nanotubes from block copolymers. Soft matter, 2005, 1, 36-43.
16) Michael A. R. Meier , Sebastianus N. H. Aerts, Bastiaan B. P. Staal, Mircea Rasa, Ulrich S. Schubert. Macromolecular Rapid Communications. 2002, 24, 1918-1924.
17) Watson Loh. Encyclopedia of Surface and Colloid Science. 2002, Marcel Dekker, Inc
18) E Rizzardo, S H Tang, G Moad, et al, Macromolecules 1998, 31, 5559.
19) bipyridine macrocycles incorporating a triethylenetraminetetraacetic acid core as ligand for lanthanide ions, Tetrahedron Letters 50 (2009) 6522-6525.
20) Mouffouk, F., Chen, L. Bioassay based on polymer micelles with femtomolar sensitivity. Anal. Biochem. 372, 140-147, 2008

## Claims

1. A nanoparticle comprising a micelle formed by
a pH sensitive amphiphilic block-copolymer, said pH sensitive amphiphilic block-copolymer being poly(ethylene glycol-b-trimethylsilyl methacrylate), and
an agent encapsulated within said micelle, said agent being a Gd³⁺-complex.

2. The nanoparticle according to claim 1, wherein said pH sensitive amphiphilic block-copolymer is capable of forming said micelle in aqueous solution through self-assembly.

3. The nanoparticle according to any of the foregoing claims, wherein said nanoparticle is conjugated to a targeting agent capable of binding specifically to a target cell.

4. The nanoparticle according to claim 1, wherein said Gd³⁺-complex is selected from the group comprising tetraaquodichloro(4,4'-ditBu-2,2'-bipyridine)gadolinium (III)chloride, and from
| | |
|---|---|
| D03A | 1,4,7,10-tetraazacyclododecane-1,4,7-trisacetic acid, |
| D03AB | 1,4,7,10-tetraazacyclododecane-1-(2,3-dihydroxy-1-hydroxymethylpropyl)-4,7,10-trisacetic acid, |
| D03A(tris-*t*-Buester) | 1,4,7-tris-*tert*-butoxycarbonylmethyl-1,4,7,10-tetraazacyclododecane, |
| DOTA | 1,4,7,10-tetraazacyclododecane-1,4,7,10-tetraacetic acid, |
| DOTA-*p*-NH2-anilide | 1,4,7,10-tetraazacyclododecane-1-(4-aminophenylcarbamoyl methyl)-4,7,10-trisacetic acid, |
| DOTA(tris-*t*-Buester) | 1,4,7,10-tetraazacyclododecane-1,4,7-tris(acetic acid-*tert*-butyl ester)-10-acetic acid, |
| DPDP | dipyridoxal diphosphate, |
| DTPA | diethylenetriaminepentaacetic acid, |
| N DTPA-BMA | {bis-[2-(carboxymethylmethylcarbamoylmethylamino) ethyl]amino} acetic acid, |
| DTPA-BMEA | [bis-(2-{carboxymethyl-[(2-methoxyethylcarbamoyl) methyl]amino} ethyl)-amino] acetic acid, |
| EOB-DTPA | {[2-(biscarboxymethylamino)-3-(4-ethoxyphenyl)propyl]-[2-(bis carboxymethylamino) ethyl]amino}acetic acid. |

5. The nanoparticle according to any of claims 4, wherein said targeting agent is selected from the group comprising (a) proteins, (b) peptides, (c) nucleic acids, (d) carbohydrates, (e) lipids, (f) hormones, and (g) chemical compounds.

6. The nanoparticle according to claim 5, wherein the
(a) proteins are selected from natural proteins, recombinant proteins, antibodies, antibody fragments, lipoproteins and protein ligands, including growth factors, cytokines, interleukins, interferons, transferin, adhesion molecules, and urokinase-type plasminogen activator; and/or
(b) peptides are selected from natural peptides and synthetic peptides; and/or
(c) nucleic acids are selected from natural or designed DNA, RNA or derivatives thereof;

7. The nanoparticle according to any of claims 3-6, wherein said target cell is selected from the group comprising cancerous cells, parasitic cells, cells infected by a virus and cells releasing TNFα.

8. The nanoparticle according to any of the foregoing claims, wherein said micelle is stable at neutral pH and disintegrates at a pH < 6.

9. The nanoparticle according to any of the foregoing claims, wherein said micelle is capable to release its content encapsulated therein either due to enzymatic reaction(s), due to the presence of metals or due to exposure to light of a defined wavelength or wavelength range.

10. The nanoparticle according to claim 9, wherein the micelle is capable to release its content after chemical modification, and/or wherein the micelle is capable to release its content due to the presence of the metals Ca and Zn.

11. A composition comprising nanoparticles according to any of the foregoing claims, wherein said agent that is encapsulated within said micelles of said nanoparticles is a Gd³⁺-complex.

12. The composition according to claim 11 or the nanoparticle according to any of claims 1-10, wherein said agent encapsulated within said micelle is a Gd³⁺-complex, for use as a magnetic resonance imaging contrast agent or for use to treat a disease or a disease that is **characterized by** a specific microenvironment within the tissue affected by such disease,
said specific microenvironment including parameters, wherein said parameters include pH being above or below physiological pH, the presence or absence of specific enzymes or metals, metal anions or cations, or a defined temperature range above or below the physiological temperature.

13. The composite or nanoparticle for use according to claim 12, wherein said composition or nanoparticle is administered to a patient in whom a magnetic resonance imaging contrast agent is intended to be used, or is administered to a patient in need thereof.

14. The composition or nanoparticle for use according to claim 12, wherein the disease to be detected and/or treated is cancer.

15. The composition or nanoparticle for use according to claim 13, wherein the patient in need is a patient having or suspected of having a disease, in particular cancer.

## Patentansprüche

1. Nanopartikel, umfassend eine Mizelle, gebildet durch
ein pH-empfindliches amphiphiles Block-Copolymer, wobei das pH-empfindliche amphiphile Block-Copolymer Poly(ethylenglycol-b-trimethylsilylmethacrylat) ist, und ein innerhalb der Mizelle verkapseltes Mittel, wobei das Mittel ein Gd³⁺-Komplex ist.

2. Nanopartikel nach Anspruch 1, wobei das pH-empfindliche amphiphile Block-Copolymer in der Lage ist, die Mizelle in wässriger Lösung mittels Selbst-Assemblierung zu bilden.

3. Nanopartikel nach einem der vorangehenden Ansprüche, wobei das Nanopartikel an ein Zielmittel konjugiert ist, das in der Lage ist, spezifisch an eine Zielzelle zu binden.

4. Nanopartikel nach Anspruch 1, wobei der Gd³⁺-Komplex ausgewählt ist aus der Gruppe, umfassend Tetraaqua dichloro(4,4'-ditBu-2,2'-bipyridin)gadolinium(III)chlorid, und aus
| | |
|---|---|
| Do3A | 1,4,7,10-Tetraazacyclododecan-1,4,7-trisessigsäure, |
| Do3AB | 1,4,7,10-Tetraazacyclododecan-1-(2,3-dihydroxy-1-hydroxymethylpropyl)-4,7,10-trisessigsäure, |
| Do3A(tris-*t*-Buester) | 1,4,7-Tris-*tert*-butoxycarbonylmethyl-1,4,7,10-tetraazacyclododecan, |
| DOTA | 1,4,7,10-Tetraazacyclododecan-1,4,7,10-tetraessigsäure, |
| DOTA-*p*-NH2-anilide | 1,4,7,10-Tetraazacyclododecan-1-(4-aminophenylcarbamoyl methyl)-4,7,10-trisessigsäure, |
| DOTA(tris-*t*-Buester) | 1,4,7,10-Tetraazacyclododecan-1,4,7-tris(Essigsäure-*tert*-butyl ester)-10-Essigsäure, |
| DPDP | Dipyridoxaldiphosphat, |
| DTPA | Diethylentriaminpentaessigsäure, |
| N DTPA-BMA | {bis-[2-(Carboxymethylmethylcarbamoylmethylamino) ethyl]amino} Essigsäure, |
| DTPA-BMEA | [bis-(2-{Carboxymethyl-[(2-methoxyethylcarbamoyl) methyl]amino} ethyl)-amino] Essigsäure, |
| EOB-DTPA | {[2-(Biscarboxymethylamino)-3-(4-ethoxyphenyl)propyl]-[2-(bis carboxymethylamino) ethyl]amino}Essigsäure. |

5. Nanopartikel nach Anspruch 4, wobei das Zielmittel ausgewählt ist aus der Gruppe, umfassend (a) Proteine, (b) Peptide, (c) Nukleinsäuren, (d) Kohlenhydrate, (e) Lipide, (f) Hormone und (g) chemische Verbindungen.

6. Nanopartikel nach Anspruch 5, wobei die
(a) Proteine ausgewählt sind aus natürlichen Proteinen, rekombinanten Proteinen, Antikörpern, Antikörperfragmenten, Lipoproteinen und Proteinliganden, einschließlich Wachstumsfaktoren, Cytokinen, Interleukinen, Interferonen, Transferrin, Adhäsionsmolekülen und Urokinase-Typ-Plasminogenaktivator; und/oder
(b) Peptide ausgewählt sind aus natürlichen Peptiden und synthetischen Peptiden; und/oder
(c) Nukleinsäuren ausgewählt sind aus natürlicher oder konstruierter DNA, RNA oder Derivaten davon.

7. Nanopartikel nach einem der Ansprüche 3-6, wobei die Zielzelle ausgewählt ist aus der Gruppe, umfassend Krebszellen, parasitische Zellen, Zellen, die mit einem Virus infiziert sind, und Zellen, die TNFα freisetzen.

8. Nanopartikel nach einem der vorangehenden Ansprüche, wobei die Mizelle bei neutralem pH stabil ist und sich bei einem pH < 6 zersetzt.

9. Nanopartikel nach einem der vorangehenden Ansprüche, wobei die Mizelle in der Lage ist, ihren darin verkapselten Inhalt freizusetzen entweder aufgrund von enzymatischer Reaktion (enzymatischen Reaktionen), aufgrund der Anwesenheit von Metallen oder aufgrund des Aussetzens gegenüber Licht mit einer definierten Wellenlänge oder Wellenlängenbereich.

10. Nanopartikel nach Anspruch 9, wobei die Mizelle in der Lage ist, ihren Inhalt nach chemischer Modifikation freizusetzen, und/oder wobei die Mizelle in der Lage ist, ihren Inhalt aufgrund der Anwesenheit der Metalle Ca und Zn freizusetzen.

11. Zusammensetzung, umfassend Nanopartikel nach einem der vorangehenden Ansprüche, wobei das Mittel, das in den Mizellen der Nanopartikel verkapselt ist, ein Gd³⁺-Komplex ist.

12. Zusammensetzung nach Anspruch 11 oder Nanopartikel nach einem der Ansprüche 1-10, wobei das Mittel, das innerhalb der Mizelle verkapselt ist, ein Gd³⁺-Komplex ist, zur Verwendung als ein Magnetresonanzbildkontrastmittel oder zur Verwendung zum Behandeln einer Krankheit oder einer Erkrankung, die durch eine spezifische Mikro-Umgebung innerhalb des durch eine solche Erkrankung betroffenen Gewebes charakterisiert ist, wobei die spezifische Mikro-Umgebung Parameter enthält, wobei die Parameter pH oberhalb oder unterhalb des physiologischen pH, die Anwesenheit oder Abwesenheit spezifischer Enzyme oder Metalle, Metallanionen oder -kationen, oder einen definierten Temperaturbereich oberhalb oder unterhalb der physiologischen Temperatur beinhalten.

13. Zusammensetzung oder Nanopartikel zur Verwendung nach Anspruch 12, wobei die Zusammensetzung oder Nanopartikel an einen Patienten verabreicht wird, in dem ein Magnetresonanzbildkontrastmittel verwendet werden soll, oder an einen hierfür bedürftigen Patienten verabreicht wird.

14. Zusammensetzung oder Nanopartikel zur Verwendung nach Anspruch 12, wobei die nachzuweisende und/oder zu behandelnde Krankheit Krebs ist.

15. Zusammensetzung oder Nanopartikel zur Verwendung nach Anspruch 13, wobei der bedürftige Patient ein Patient ist, der einen Krankheit hat oder von dem vermutet wird, dass er sie hat, insbesondere Krebs.

## Revendications

1. Nanoparticule comprenant une micelle formée par un copolymère séquencé amphiphile sensible au pH, ledit copolymère séquencé amphiphile sensible au pH étant un poly(éthylèneglycol-b-méthacrylate de triméthylsilyle), et
un agent encapsulé au sein de ladite micelle, ledit agent étant un complexe de Gd³⁺.

2. Nanoparticule selon la revendication 1, dans laquelle ledit copolymère séquencé amphiphile sensible au pH est capable de former ladite micelle dans une solution aqueuse par auto-assemblage.

3. Nanoparticule selon l'une quelconque des revendications précédentes, dans laquelle ladite nanoparticule est conjuguée à un agent de vectorisation capable de se lier spécifiquement à une cellule cible.

4. Nanoparticule selon la revendication 1, dans laquelle ledit complexe de Gd³⁺ est choisi dans le groupe comprenant le chlorure de tétraaquodichloro-(4,4'-ditBu-2,2'-bipyridine)gadolinium (III), et parmi
l'acide 1,4,7,10-tétraazacyclododécane-1,4,7-trisacétique D03A,
l'acide 1,4,7,10-tétraazacyclododécane-1-(2,3-dihydroxy-1-hydroxyméthylpropyl)-4,7,10-trisacétique D03AB,
le 1,4,7-tris-tert-butoxycarbonylméthyl-1,4,7,10-tétraazacyclododécane D03A(tris-ester de t-Bu),
l'acide 1,4,7,10-tétraazacyclododécane-1,4,7,10-tétraacétique DOTA,
l'acide 1,4,7,10-tétraazacyclododécane-1-(4-amino-phénylcarbamoylméthyl)-4,7,10-trisacétique DOTA-p-NH2-anilide,
l'acide 1,4,7,10-tétraazacyclododécane-1,4,7-tris-(acétate de *tert*-butyle)-10-acétique DOTA(tris-ester de t-Bu),
le diphosphate de dipyridoxal DPDP,
l'acide diéthylènetriaminepentaacétique DTPA,
l'acide {bis-[2-(carboxyméthylméthylcarbamoyl-méthylamino)éthyl]amino}acétique N DTPA-BMA,
l'acide [bis-(2-{carboxyméthyl-[(2-méthoxyéthyl-carbamoyl)méthyl]amino}éthyl)-amino]acétique DTPA-BMEA,
l'acide {[2-(biscarboxyméthylamino)-3-(4-éthoxy-phényl)propyl]-[2-(biscarboxyméthylamino)éthyl]amino}-acétique EOB-DTPA.

5. Nanoparticule selon la revendication 4, dans laquelle ledit agent de vectorisation est choisi dans le groupe comprenant (a) les protéines, (b) les peptides, (c) les acides nucléiques, (d) les glucides, (e) les lipides, (f) les hormones et (g) les composés chimiques.

6. Nanoparticule selon la revendication 5, dans laquelle
(a) les protéines sont choisies parmi les protéines naturelles, les protéines recombinantes, les anticorps, les fragments d'anticorps, les lipoprotéines et les ligands protéiques, y compris les facteurs de croissance, les cytokines, les interleukines, les interférons, la transférine, les molécules d'adhérence, et un activateur du plasminogène de type urokinase ; et/ou
(b) les peptides sont choisis parmi les peptides naturels et les peptides de synthèse ; et/ou
(c) les acides nucléiques sont choisis parmi un ADN naturel ou conçu, un ARN naturel ou conçu ou leurs dérivés.

7. Nanoparticule selon l'une quelconque des revendications 3 à 6, dans laquelle ladite cellule cible est choisie dans le groupe comprenant les cellules cancéreuses, les cellules parasites, les cellules infectées par un virus et les cellules libérant du TNFα.

8. Nanoparticule selon l'une quelconque des revendications précédentes, dans laquelle ladite micelle est stable à pH neutre et se désintègre à un pH < 6.

9. Nanoparticule selon l'une quelconque des revendications précédentes, dans laquelle ladite micelle est capable de libérer le contenu qu'elle encapsule du fait d'une ou de plusieurs réactions enzymatiques, du fait de la présence de métaux ou du fait d'une exposition à une lumière ayant une longueur d'onde définie ou une plage définie de longueurs d'onde.

10. Nanoparticule selon la revendication 9, dans laquelle la micelle est capable de libérer son contenu après une modification chimique, et/ou dans laquelle la micelle est capable de libérer son contenu du fait de la présence des métaux Ca et Zn.

11. Composition comprenant des nanoparticules selon l'une quelconque des revendications précédentes, dans laquelle ledit agent qui est encapsulé au sein desdites micelles desdites nanoparticules est un complexe de Gd³⁺.

12. Composition selon la revendication 11 ou nanoparticule selon l'une quelconque des revendications 1 à 10, dans laquelle ledit agent encapsulé au sein de ladite micelle est un complexe de Gd³⁺, pour son utilisation comme agent de contraste pour imagerie par résonance magnétique ou pour son utilisation pour traiter une maladie ou une maladie qui est **caractérisée par** un microenvironnement spécifique au sein du tissu affecté par une telle maladie,
ledit microenvironnement spécifique présentant des paramètres, dans lequel lesdits paramètres comprennent un pH supérieur ou inférieur au pH physiologique, la présence ou l'absence d'enzymes ou de métaux, anions métalliques ou cations métalliques spécifiques, ou une plage de températures définie supérieure ou inférieure à la température physiologique.

13. Composite ou nanoparticule selon la revendication 12, dans laquelle ladite composition ou nanoparticule est administrée à un patient chez qui il est prévu d'utiliser un agent de contraste pour imagerie par résonance magnétique, ou est administré à un patient en ayant besoin.

14. Composition ou nanoparticule pour son utilisation selon la revendication 12, dans laquelle la maladie à détecter et/ou à traiter est un cancer.

15. Composition ou nanoparticule pour son utilisation selon la revendication 13, dans laquelle le patient en ayant besoin est un patient ayant ou suspecté d'avoir une maladie, en particulier un cancer.
